# EUROPEAN PATENT APPLICATION

(11) **EP 2 363 487 A2**
(43) Date of publication of application: **07.09.2011**
(21) Application number: 10011286.1
(22) Date of filing: 23.12.2002
(51) Int. Cl.: C12N 15/33, C12N 15/864, C07K 14/015, A61K 38/00, A61K 48/00

(54) **A library of modified structural genes or capsid modified particles useful for the identification of viral clones with desired cell tropism**

(30) Priority: 21.12.2001 US 344131 P; 07.03.2002 US 362349 P; 30.08.2002 US 407116 P
(62) Divisional of application: 02787962.6
(71) Applicant: Medigene AG, 82152 Planegg/Martinsried (DE)
(72) Inventor: Perabo, Luca, 13347 Berlin (DE); Büning, Hildegard, 50933 Köln (DE); Enssle, Jörg, 72649 Wolfschlugen (DE); Ried, Martin, 82319 Starnberg (DE); Hallek, Michael, 50935 Köln (DE); Huttner, Nadja, 81241 München (DE)
(74) Representative: Bösl, Raphael Konrad

(57) **Abstract**

The present invention relates to libraries containing modified parvovirus cap genes useful for the identification of parvovirus capsids able to transduce predefined cell types as well as to methods for the production thereof.

## Description

The present invention relates to libraries containing modified parvovirus cap genes useful for the identification of parvovirus capsids able to transduce predefined cell types as well as to methods for the production thereof.

The control of the tropism of the vector (retargeting) represents a critical concern in the development of viral gene transfer systems for gene therapy: to allow efficient transfer of the therapeutic genes to the target cells and to avoid transduction of undesired cell types. Efforts to achieve these goals led to the description of several approaches aimed to provide virions with the ability to interact with specific cellular receptors. One of these approaches includes the coupling of viral particles to receptor-binding molecules, which results in retargeted vectors with improved specificity. A big disadvantage of this technology is however that these retargeting molecules could detach from the capsids, restoring the natural tropism of the virions.

A different approach consists in the genetic modification of the viral capsid or envelope proteins by site directed mutagenesis, mostly by insertional mutagenesis.

For all these procedures, the critical step is the choice of functional retargeting molecules. This problem has been adressed in several studies by taking advantage of the phage display technology to screen large number of peptides for desired binding ability to specific receptors or cell types. However, the introduction of foreign molecules at the level of the external structure of the vector is likely to disturb the integrity of the viral particles, resulting in low titer or inefficient viral preparations. Moreover, these ligand peptides could completely or partially lose their affinity for the aimed receptor once introduced in the architecture of the vector. An elegant solution to this problem has been proposed for adenoviral vectors (Pereboev A. et al. (2001) J Viro 75(15), 7107-13). In this publication the expression of the Ad. fiber knob on the surface of pJuFo-phages is described, which allows the display of polypeptide randomized sequences in a context that mimics the microenvironment of the destination vector. However, a physiological limitation of phage-based libraries cannot be overcome: the molecules can be selected exclusively on the basis of their binding ability, while optimization of the uptake and processing of the viral particles inside the cell cannot be pursued.

Parvoviruses and especially the Adeno-associated virus (AAV) have received increasing attention as a vector for gene therapy because of their non-pathogenicity, their low immunogenicity, and their ability to infect both dividing and non-dividing cells and to facilitate long term expression of the therapeutic genes. Furthermore, AAV is able to integrate site specifically into the genome of the infected cell without impairing any cellular function.

A great disadvantage of the use of parvoviruses and other viruses in gene therapy is the fact that these viruses are only able to efficiently transduce specific cell types, while other cell types are resistant against a parvovirus infection. Especially, AAV2 is only able to transduce cells having heparan sulfate proteoglycan (HSPG) on their surface (Girod A. et al.(1999) Nature Medicine 5-9, 1052-56). As mentioned above, the control of the viral tropism is crucial for the effective transfer of the therapeutic genes to the target cells and for the prevention of a transduction of undesired cell types.

In the publication Girod A. et al.((1999) Nature Medicine 5-9, 1052-56) it was recently demonstrated that it is possible to modify the external structure of Adeno-associated virus in order to provide the capsid with the ability to interact with cellular receptors that are not recognized by the wild type virus. This procedure can be performed by insertion of a ligand sequence at the level of a specific site of the viral capsid protein, that is presented on the external surface of the capsid. As one suitable locus, the amino acidic position 587 of the major viral capsid protein (VP1) of AAV2 was identified. The insertion at this site of the L14 sequence, an RGD motif containing peptide. (a portion of the laminin fragment P1); provided the so obtained mutant AAV (L 14-AAV) with the ability to infect B16F10 cells. The B16F10 cells express an integrin and are, due to the lack of expression of heparan sulfate proteoglycans at the outer cellular membrane, resistant to infection with wtAAV2.

To date, no system exists which allows the fast identification of mutated viruses, especially parvoviruses which are able to infect one cell type but are unable to infect other cell types. Such a system is highly required for obtaining cell type specific parvoviruses to be used in gene therapy.

Consequently, it is the object of the present invention to provide systems for the identification of virus, especially parvovirus mutants which are able to infect other cell types than the corresponding wild type viruses. Furthermore, it is the object of the present invention to provide methods for the preparation of such systems.

The present invention relates therefore in a first subject matter to a method for the production of a library of nucleic acids comprising a multiplicity of expressible structural genes from at least one eukaryotic virus, comprising the steps of:
a) providing a set of nucleic acids, each encoding at least one structural gene from a eukaryotic virus and comprising a suitable packaging sequence, and
b) inserting a first insert (1) into the structural gene.

According to the present invention, the expression "structural gene" relates to a gene encoding one or more proteins, preferably structural proteins of a viral capsid either of a non-enveloped or an enveloped virus or to gene encoding one or more proteins, preferably structural proteins of a viral shell of enveloped viruses.

The invention further relates to a library of nucleic acids comprising a multiplicity of expressible structural genes from at least one eukaryotic virus, obtainable by the above method.

The library of the invention contains a multiplicity of nucleic acids with different structural genes which may be expressed in order to form infectious viral particles with a tropism for different cell types. Given a certain cell type, it is therefore possible to screen the library for mutant viruses, especially parvoviruses which are able to infect that specific cell type.

According to the invention, the term "capsid protein" means a protein encoded by a cap gene, whereas "functional capsid protein" means a capsid protein of a virus able to infect at least one host cell.

In case of AAV, the capsid protein may be VP 1, VP2 or VP3, for other parvoviruses names and numbers of the capsid proteins may differ.

According to the invention, the term "packaging sequence" means a cis-acting nucleic acid sequence that mediates the packaging of a nucleic acid into a viral capsid. For parvoviruses e.g. it is known in the art, that the so called "inverted terminal repeats" (ITRs) that are located at the 5' and 3' end of the linear viral genome have this function.

According to a preferred embodiment, the structural genes are from an enveloped virus such as a retrovirus, lentivirus, herpes virus, e.g. HSV1, HSV2, EBV, Varizella zoster virus, human herpes virus 1, 2, 3, 4, 7 or 8.

According to another preferred embodiment, the structural genes are cap genes, preferably from a non-enveloped virus such as parvovirus or adenovirus. In this case, the cap genes encode for one or more capsid proteins

According to the present invention, the expression "cap gene" relates to a gene encoding one or more proteins of a viral capsid either of a non-enveloped or an enveloped virus.

More preferably, the cap genes are from a parvovirus selected from the group consisting of Adeno-associated Virus (AAV), Canine Parvovirus (CPV), MVM, B19, H1, AAAV (Avian AAV) or GPV (goose parvovirus).

Most preferred, the cap genes are from an AAV, e.g. AAV 1, AAV2, AAV3, AAV4, AAV5 or AAV6.

In a preferred embodiment, the library obtainable by the method of the invention has a multiplicity of viral mutants that is greater than 10², preferably greater than 10⁵, especially greater than 10⁶ and, in another preferred embodiment, a multiplicity of expressible structural genes, preferably cap genes that is greater than 10², preferably greater that 10⁵, especially greater that 10⁶.

In a preferred embodiment, the set of nucleic acids is derived from one nucleic acid. In this case, the library is constituted of a multiplicity of nucleic acids which are, apart from the insert, almost identical. However, it is also included within the present invention that the set of nucleic acids may be derived from different nucleic acids encoding structural genes. In this case, it is preferred that the nucleic acids are derived from one virus.

According to a preferred embodiment, one insert (1) is inserted into the structural gene. However, it is also included within the present invention that more than one inserts (1) are inserted. Preferably two, three or up to six inserts (1) are inserted into the structural gene. The insertion may, dependent on the insertion site, lead to an amino acid insertion in one or more structural proteins, preferably capsid proteins, e. g. VP1, VP2 and/or VP3 in the case of AAV. In this context it should be noted that, in the case of parvoviruses, parvovirus capsid proteins are encoded by only one cap gene by overlapping reading frames.

In a preferred embodiment of the present invention a sequence of the structural gene is removed by inserting insert (1).

In a further preferred embodiment, the removed sequence comprises or is part of an insert (2) inserted into the structural gene before step (a).

As a preferred embodiment the method further comprises an initial step, wherein the structural gene is modified to render the structural gene non-functional. This can be achieved i.e. by inserting insert (2). By a replacement of insert (2) with insert (1) in step a) of the method, potentially functional structural genes will be formed. In the case of parvoviruses, this additional step leads to a reduced number of parvovirus virions that have a capsid built from viral capsid proteins not encompassing insert (1) and therefore enables the formation of libraries with high titers.

Consequently, in a more preferred embodiment, insert (2) prevents the formation of a functional structural protein, preferably a capsid protein, preferably by containing a stop codon. Furthermore, insert (2) may shift the open reading frame or may introduce additional amino acids which disturb the formation of functional capsids or their infection biology at any further step.

In a preferred embodiment, insert (1) and/ or insert (2) contains further at least two restriction sites, preferably one at its 3'-end and one at its 5'-end.

According to a preferred embodiment, insert (2) is at least partially replaced by insert (1), whereby the prevention of the formation of functional capsids is - at least for some capsids - abolished, in a preferred embodiment by removing the stop codon.

According to a preferred embodiment, the number of nucleotides of insert (1) and/or insert (2), preferably of insert (1) and insert (2), is three or a multiple of three.

According to a preferred embodiment, insert (1) is inserted at a region of the cap gene encoding amino acids on the surface of the capsid protein.

In preferred embodiments, the virus is AAV2 and insert (1) is inserted after a nucleic acid corresponding to a site within the first amino terminal amino acids 1 to 50, or corresponding to amino acid positions 261, 381, 447, 534, 573, and/or 587 of the capsid protein VP1, preferably corresponding to amino acid position 447 or 587.

In additional preferred embodiments, insert (1) is inserted in nucleic acids corresponding to the adjacent 5 amino acid of the above indicated insertion sites, as these amino acid stretches represent loops of the AAV2-capsid and therefore are located on the surface of the capsid protein. It is possible for the person skilled in the art to identify corresponding loops and insertion site for other parvoviruses by known techniques such as sequence alignment, three-dimensional structure analysis, protein folding, hydrophobicity analysis (Girod A et al, 1999 supra).

Especially preferred insertion sites are within the stretches consisting of amino acids (aa)
i) aa 261-270, especially aa 269,
ii) aa 324-331,
iii) aa 380-383, especially aa 381,
iv) aa 447-460, especially aa 447 and 451-460,
v) aa 484-503, preferably aa 484-499, especially aa 484, 487 or aa 494-499,
vi) aa 507-514, especially aa 507, 509 or 514,
vii) aa 527-534, especially 527-529, 532 or 534,
viii) aa 548-556;
ix) aa 572-575, especially 573,
x) aa 581-595, especially 585, 587, 588 or 594.

The numbering of amino acids relates to the VP1 protein of AAV2 (Girod A et al, 1999 supra).

Stretches v) and vi) are especially preferred.

The insert (1) inserted into the nucleic acid my be identical for all structural genes where an insert (1) is inserted. However, it is preferred that insert (1) is different or at least potentially different for all structural genes where an insert (1) is inserted.

In preferred embodiments, insert (1) is randomly or partially randomly generated. This means that the insert (1) introduced in one structural gene is potentially different from the insert (1) inserted in another structural gene, although it is theoretically possible that two inserts (1) are identical.

When the inserted nucleic acid sequences are randomly generated, in a preferred embodiment the codons NNN, NNB or NNK (N=A,C,G or T; B=C,G or T; K=G or T) are used. Furthermore, the inserted nucleic acid sequences may be partially randomly generated, especially using codons with one, two or three fixed nucleotides. The length of such insertions may be preferably at least 3 nucleotides, preferably at least 9, especially at least 18 nucleotides.

In a preferred embodiment, insert (1) may contain, in addition to the randomly or partially randomly generated sequences, a further stretch of at least one codon upstream and/or downstream of the randomized or partially randomized nucleic acid sequences, preferably of one or two or three codons coding for Ala, Gly, Leu, Ile,Asp and/or Arg, especially an insertion of three codons for Ala-upstream and two codons for Ala downstream of the randomized or partially randomized nucleic acid sequences.

In a preferred embodiment, insert (1) does not contain any stop codons. This can be achieved by not having an A or G at the third position of the codons of insert (1).

Furthermore, the library obtainable by the method of the invention may have the features as defined below for the library of nucleic acids comprising a multiplicity of expressible structural genes from at least one eukaryotic virus.

In a further subject matter, this invention relates to a library of nucleic acids comprising a multiplicity of expressible structural genes, preferably cap genes, from an eukaryotic virus, preferably of a parvovirus, especially dependoviruses such as Adeno-associated virus or a canine parvovirus (CPV) as well as autonomous parvoviruses such as H1, MVM (minute virus of mice) or B19, AAAV or GPV.

Said library of nucleic acids may encode eucaryotic viruses with a multiplicity of modifications of the virion's external structure and/or of the corresponding (encoding) genetic information. The library has a preferred multiplicity of viral mutants that is greater than 10², preferably greater that 10⁵, especially greater that 10⁶_{.}

In a further embodiment, the library of nucleic acids has a multiplicity of expressible structural genes, preferably cap genes that is greater than 10², preferably greater that 10⁵, especially greater that 10⁶.

The library may be in the form of a linear nucleic acid, a plasmid, a viral particle or a viral vector, e.g. a recombinant AAV, Adenovirus or Herpes Simplex Virus vector.

In a preferred embodiment, the nucleic acid may additionally comprise packaging sequences (e.g. AAV ITRs) and expressible genes providing necessary functions for replication and packaging of virions (e.g. non-structural genes for parvoviruses such as AAV rep gene).

These sequences, genes or functions may be provided in cis (meaning on the same construct as the packaging sequences and the capsid proteins encoding genes) or in trans (meaning on a different construct) However, the packaging sequences must be provided in cis.

In a preferred embodiment, the nucleic acid is DNA.

In preferred embodiments, the cap gene as well as packaging sequences such as ITRs and genes providing necessary functions for replication and packaging of virions, such as the rep gene, are derived from parvoviruses, preferably from dependoviruses such as AAV or CPV, especially from one of the AAV serotypes from the group comprising AAV1, AAV2, AAV3, AAV4, AAV5 and AAV6 or from autonomous parvoviruses such as H1, MVM, B19, AAAV or GPV.

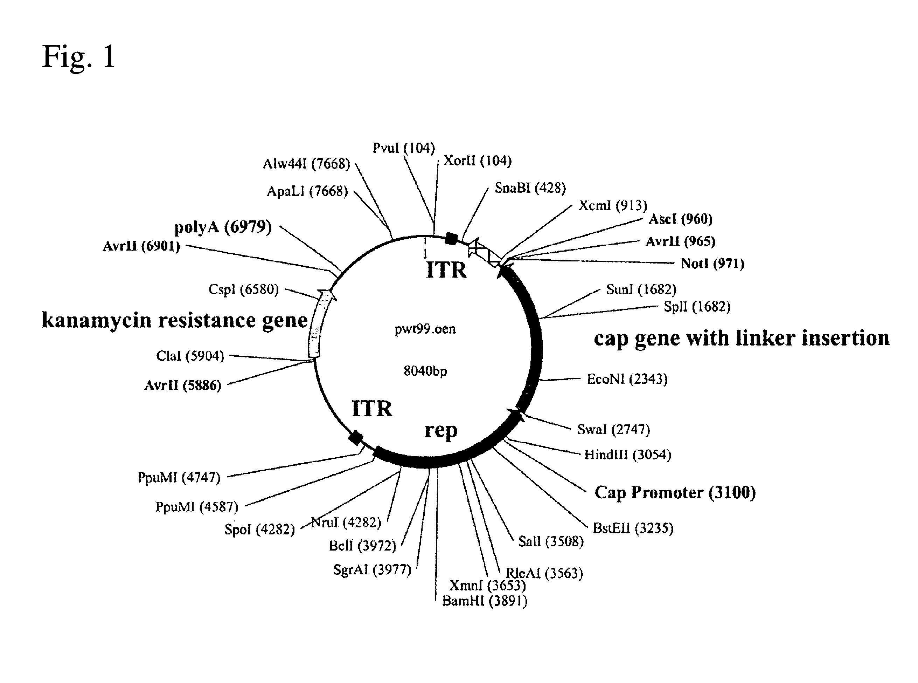

Another preferred embodiment relates to a library, wherein the AAV cap gene is derived from the AAV cap gene encoded in plasmid pWT99oen (see Fig. 1 and Example 1).

The multiplicity of nucleic acid sequences are inserted into at least one site of the structural gene, preferably the cap gene, wherein the number of inserted nucleotides is three or a multiple of three. According to preferred embodiments, the multiplicity of nucleic acid sequences are inserted into one, two or three sites of the structural gene, preferably the cap gene.

The inserted nucleic acid sequences are preferably randomly generated, especially using NNN codons, NNB codons or NNK codons (N=A,C,G or T;B=G,G or T; K=G or T). Furthermore, the inserted nucleic acid sequences may be partially randomly generated, especially using codons with one, two or three fixed nucleotides.

In a further preferred embodiment, a second or further insert (1) may contain non randomized codons for amino acid stretches of choice. This has the advantage that one can simultaneously screen for expressible structural genes with a wanted property by inserting a randomized insert (1) and one or more further inserts (1) at different sites to change the properties of such an expressible structural gene.

For example, if one has already identified an insert (1) that codes for a peptide and leads to a retargeted vector or a vector with other wanted properties, one can use this insert at a specific site and use a randomized insert (1) at another site to screen for a vector with other, enhanced properties. This procedure can be repeated for several or all known potential insertion sites.

Furthermore, one can combine the insertion of a randomized insert (1) with the insertion of further fixed inserts (1) preferably at known or presumed epitopes to change the immunogenicity of the vector. Within the scope of this invention it was shown that either by using a randomized insert (1) and screening for a vector with an increased infectivity or specificity for a specific cell type (rAAV-587/Mec, example 7) or by inserting a fixed insert (rAAV-587/L14, example 7) one can abolish or reduce the neutralizing effects of antibodies. Therefore, the invention can also be used to make or screen for vectors that have a reduced binding to antibodies (monoclonal or polyclonal antibodies/sera) and/or to have the ability to escape neutralizing antibodies and therefore are able to escape from an immune response in a patient.

In a preferred embodiment, the length of such insertions is at least 3 nucleotides, preferably at least 9, especially at least 18 nucleotides.

The inserted nucleic acid sequences may have been inserted using standard restriction endonucleases, recombination systems, e.g. the gateway or the cre/lox recombination system or polymerase chain reaction techniques, e.g. using degenerated primers.

The inserted nucleic acid sequences shall lead to an insertion of amino acids into at least one viral capsid protein, i.e. in the case of AAV into VP1, VP2 and/or VP3 structural protein, preferably at a site that is located on the surface of the capsid of the virion.

The inserted nucleic acid sequences may be inserted at any site within the first amino terminal amino acids 1 to 50 of VP1, after corresponding amino acid positions 261, 381, 447, 534, 573, and/or 587 of VP1, preferably after amino acid position 447 or 587. The numbering of the amino acids relates to the position within VP1. For the avoidance of doubt, corresponding sites of VP2 and VP3 of course have a different number.

For AAV, this means that the inserted nucleic acid sequences may be inserted after a nucleic acid corresponding to any site within the first amino terminal amino acids 1 to 50 of VP1, or corresponding to amino acid positions 261, 381, 447, 534, 573, and/or 587 of VP1, preferably to amino acid position 447 or 587. The numbering of the amino acids relates to the position within VP1. For the avoidance of doubt, corresponding sites of VP2 and VP3 of course have a different number.

In additional preferred embodiments, insert (1) is inserted in nucleic acids corresponding to the adjacent 5 amino acid of the above indicated insertion sites, as these amino acid stretches represent loops of the AAV2-capsid and therefore are located on the surface of the capsid protein. It is possible for the person skilled in the art to identify corresponding loops and insertion site for other parvoviruses by known techniques such as sequence alignment, three-dimensional structure analysis, protein folding, hydrophobicity analysis (Girod A et al, 1999 supra)

The cap genes may according to preferred embodiments in addition have at least one further mutation being for example at least one point mutation, at least one internal deletion, insertion and/or substitution of one or several amino acids or at least one N- or C-terminal deletion, insertion and/or substitution of one or several amino acids, or a combination of these mutations, preferably a mutation inhibiting heparansulfate proteoglycan, integrins and/or Fibroblast Growth Factor Receptor (FGFR) binding. These additional specific mutations are especially advantageous, since they reduce the infectivity of the virion for a large number of its natural host cells.

Such further mutations can be used for an additional modification of infectivity of the Cap protein/virion, for a reduction of an infection not mediated by AAV e.g. by reducing or abolishing binding for cellular receptors, or for a changed immunogenicity of the Cap protein/virion by reducing or abolishing the affinity to antibodies especially escaping from neutralizing antibodies.

Furthermore, such cap genes may have a further constant insertion of at least one codon upstream and/or downstream of the insertion sites of the randomized nucleic acid sequences, preferably of one or two or three codons coding for Ala, Gly, Leu, Ile, Asp and/or Arg, especially an insertion of three Ala upstream and two Ala downstream of the insertion site.

Furthermore this invention relates to a library of virions, especially parvovirus virions, with capsid protein modifications.

In a preferred embodiment of the invention, the library of virions contains particles containing the genetic information necessary to generate viral progeny.

A preferred embodiment is a library of said virions, where each particle contains the genetic information necessary to generate viral progeny.

In a particularly preferred embodiment of the invention said library of virions is generated by using any of the above mentioned nucleic acids.

A further embodiment of this invention is a cap gene that comprises at least one recombination site within the cap gene, e.g. for the Gateway or cre/lox system, preferably after amino acid position 587 of VP1 wherein the inserted nucleic acid sequences are inserted at any site within the first amino-terminal amino acids 1 to 50 of VP1, after corresponding amino acid positions 261, 381, 447, 534, 573, and/or 587 of VP1, preferably after amino acid position 447 or 587.

Especially preferred insertion sites are within the stretches consisting of amino acids (aa)
i) aa 261-270, especially aa 269,
ii) aa 324-331,
iii) aa 380-383, especially aa 381,
iv) aa 447-460, especially aa 447 and 451-460,
v) aa 484-503, preferably aa 484-499, especially aa 484, 487 or aa 494-499,
vi) aa 507-514, especially aa 507, 509 or 514,
vii) aa 527-534, especially 527-529, 532 or 534,
viii) aa 548-556,
ix) aa 572-575, especially 573,
x) aa 581-595, especially 585, 587, 588 or 594.

The numbering of amino acids relates to the VP1 protein of AAV2 (Girod A et al, 1999 supra).

Stretches v) and vi) are especially preferred.

This means that a further subject matter of this invention is a cap gene that comprises at least one recombination site within the cap gene, preferably for the Gateway or cre/lox system. For AAV, the recombination site may be inserted after a nucleic acid corresponding to any site within the first amino terminal amino acids 1 to 50 of VP1, or corresponding to amino acid positions 261, 381, 447, 534, 573, and/or 587 of VP1, preferably to amino acid position 447 or 587. The numbering of the amino acids relates to the position within VP1. For the avoidance of doubt, corresponding sites of VP2 and VP3 of course have a different number. This cap gene of the invention can be used as starting material for the method of the invention for producing a parvovirus library.

Furthermore the cap gene may comprise at least one endonuclease restriction site or polylinker that is not present in the respective wildtype gene site useful for the insertion at any site within the first amino-terminal amino acids 1 to 50 of VP1, after corresponding amino acid positions 261, 381, 447, 534, 573, and/or 587 of VP1, preferably after amino acid position 447 or 587.

This means that the cap gene may comprise at least one endonuclease restriction site or polylinker that is not present in the respective wildtype gene. In the case of AAV2, the restriction site may be inserted after a nucleic acid corresponding to any site within the first amino-terminal amino acids 1 to 50 of VP1, or corresponding to amino acid positions 261, 381, 447, 534, 573, and/or 587 of VP1, preferably to amino acid position 447 or 587.

In a preferred embodiment, the endonuclease restriction site or polylinker may further contain a stop codon.

This will provide cap genes that do not contain an insertion with a translation stop signal that will lead to defective capsid proteins and therefore to no wild type virus production during the generation of the library.

In a preferred embodiment, the cap gene of the invention may further have at least one mutation, preferably at least one point mutation, at least one internal deletion, insertion and/or substitution of one or several amino acids or at least one N- or C-terminal deletion, insertion and/or substitution of one or several amino acids, or a combination of these mutations.

Furthermore, such cap gene may have a further constant insertion of at least one codon upstream and/or downstream of the insertion sites of the randomized nucleic acid sequences, preferably of one or two or three codons coding for Ala, Gly, Leu, Ile, Asp and/or Arg, especially an insertion of three Ala upstream and two Ala downstream of the insertion site.

In a further preferred embodiment, the cap gene may contain non randomized codons for amino acid stretches of choice. This has the advantage that one can simultaneously screen for expressible structural genes with a wanted property by inserting a randomized insert and one or more further inserts at different sites to change the properties of such an expressible structural gene.

For example, if one has already identified an insert that codes for a peptide and leads to a retargeted vector or a vector with other wanted properties, one can use this insert at a specific site and use a randomized insert at another site to screen for a vector with other, enhanced properties. This procedure can be repeated for several or all known potential insertion sites.

Furthermore, one can combine the insertion of a randomized insert with the insertion of further fixed inserts preferably at known or presumed epitopes to change the immunogenicity of the vector. Within the scope of this invention it was shown that either by using a randomized insert and screening for a vector with an increased infectivity or specificity for a specific cell type (rAAVö-587/Mec, example 7) or by inserting a fixed insert (rAAV-587/L14, example 7) one can abolish or reduce the neutralizing effects of antibodies. Therefore, the invention can also be used to make or screen for vectors that have a reduced binding to antibodies (monoclonal or polyclonal antibodies/sera) and/or to have the ability to escape neutralizing antibodies and therefore are able to escape from an immune response in a patient.

Therefore, in a most preferred embodiment, the cap gene of the invention contains an insert with
a) a restriction site or a recombination site;
b) one or more codons encoding further amino acids, preferably Ala, Gly, Leu, Ile, Asp and/or Arg; and
c) a sequence stretch preventing formation of functional capsid proteins, preferably a stop codon.

A further subject matter of this invention is the nucleic acid encoding a cap gene with a sequence of the plasmid pWT99oen (Fig 1, sequence given and Example 1).

A further subject matter of this invention is a nucleic acid encoding a cap gene, wherein such cap gene has an insertion leading to additional amino acids comprising an RGD or DDD motif, preferably an RGDXP or DDDXP motif, especially an RGD motif that is not present in human proteins, excluding the insertion

AGTFALRGDNPQG. Such cap gene may have the insertion corresponding to RGDXXXX, RGDXPXX, DDDXPXX, RGDAVGV or RGDTPTS, GKLFVDR, RDNAVVP, GENQARS, RSNGVVP, RSNAVVP or NSVRAPP.

The invention further relates to Cap proteins encoded by the above cap genes of the invention.

Another embodiment of this invention is a nucleic acid encoding a cap gene, wherein such cap gene has an insertion of the nucleotidic sequence GANGANNACNNNNCNANNANN (N = A,C,G or T) or an insertion comprising that sequence.

The inserted nucleic acid sequences may be inserted at any site corresponding to the first amino-terminal amino acids 1 to 50 of VP1, after corresponding amino acid positions 261, 381, 447, 534, 573, and/or 587 of VP1, preferably after amino acid position 447 or 587.

This means that in the case of AAV2, the nucleic acid of the invention may be inserted after a nucleic acid corresponding to any site within the first amino-terminal amino acids 1 to 50 of VP1, or corresponding to amino acid positions 261, 381, 447, 534, 573, and/or 587 of VP1, preferably to amino acid position 447 or 587.

In a preferred embodiment, the cap gene of the invention has at least one mutation leading to preferably at least one point mutation, at least one internal deletion, insertion and/or substitution of one or several amino acids or at least one N- or C-terminal deletion, insertion and/or substitution of one or several amino acids, or a combination of these mutations.

In a further preferred embodiment, such cap gene may have a further constant insertion of at least one codon upstream and/or downstream of the insertion sites of the randomized nucleic acid sequences, preferably of one or two or three codons coding for Ala, Gly, Leu, Ile, Asp and/or Arg, especially an insertion of three Ala upstream and two Ala downstream of the insertion site.

The invention further relates to the use of a nucleic acid-of the invention encoding a cap gene for the preparation of a library of nucleic acids comprising a multiplicity of expressible cap genes from at least one eukaryotic virus, preferably a parvovirus.

Further embodiments of this invention are vector constructs, bacteria or cells comprising any of the previously mentioned cap genes or constructs.

A further embodiment of this invention is a method for the selection of a recombinant virion with an increased infectivity or specificity for a specific cell type comprising the steps of
i) providing at least one first cell with a vector construct comprising at least one nucleic acid from the library of the invention together with a second nucleic acid (especially packaging sequences such as AAV ITRs and one or more genes providing non-structural functions such as replication and packaging, for example functions of an AAV Rep protein) necessary for the packaging of a virion;
ii) providing such first cell with necessary cellular, viral, physical and/or chemical helper functions for the packaging of virions if necessary;
iii) incubating such first cell under suitable conditions for the packaging of virions and collecting produced virions by such first cell;
iv) infecting at least one second cell with such collected virions;
v) providing such second cell with necessary cellular, viral, physical and/or chemical helper functions for the packaging of a virion;
vi) incubating such second cell under suitable conditions for the packaging of virions and collecting produced virions by such second cell;
whereas steps iv) to vi) can be repeated several times.

The non-structural functions as for example the Rep protein can be provided in cis or in trans.

Furthermore, the first cell and the second cell can be of the same kind or type.

A further embodiment of the invention is a method for the selection of a recombinant virion with an increased infectivity or specificity for a specific cell type, that at the same time has a reduced or no-infectivity for another cell type. To achieve such negative selection the above method additionally comprises the steps
vii) infecting at least one third cell (that shall not be infected) with the collected virions, whereas such third cell is not permissive fur such virions, and
viii) collecting the virions that did not infect such third cell.

Using these additional steps virions that are able to infect such third cells enter the cells but do not replicate within these cells due to the non-permissiveness of the cells. Therefore such virions are depleted from the library. Also these steps can be repeated on the same or different cell types.

Non-permissive cells can be obtained for both helper dependent and helper independent virions by not providing such third cell with all necessary cellular, viral, physical and/or chemical functions for the packaging of virions. One can also use drugs that inhibit viral replication and/or packaging but not infection such as acilovir for HSV. Furthermore one can use virions that have been made replication incompetent by mutations so that a cell has to provide a certain function to be permissive again.

Furthermore, this invention relates to a method for the identification of a mutant cap gene leading to virions having an increased infectivity or specificity for a specific cell type comprising the previous steps and in addition the step of cloning the nucleic acid of the cap gene(s) of the virion.

In a preferred embodiment, the method for selection of a recombinant virion invention further includes a step for the additional selection of virions, preferably an affinity binding step of virions (e.g. to a known receptor or binding motif that may be coupled to beads or a resin, for example by an affinity chromatography), an ion exchange chromatography step (to improve purifaction capabilites of such virions) or an immuno-selection step (to circumvent potential immune reactions from patients, e.g. by immuno depletion with antibodies).

In a further preferred embodiment, the invention relates to a method for the selection of a receptor binding motif comprising the steps as defined above, wherein such second cell is permissive for the respective vector.

Such receptor may be expressed recombinantly, preferably over-expressed by known recombinant technologies.

A further embodiment of the invention is a method for the in vivo selection of a recombinant virion capable of infecting a specific cell type comprising the steps of
(i) providing at least one first cell with a vector construct comprising at least one nucleic acid from the previously described library together with packaging sequences such as AAV ITRs and one or more genes providing necessary non-structural functions such as replication and packaging, for example of an AAV Rep protein for the packaging of a virion;
(ii) providing such first cell with necessary cellular or viral helper functions for the packaging of a virion if necessary;
(iii) incubating such first cell under suitable conditions for the packaging of virions, preferably AAV, and collecting produced virions, preferably AAV, by such first cell;
(iv) infecting an animal with such virions.

This method can be used for the identification of a mutant cap gene leading to virions having an increased infectivity or specificity for a specific cell type by addition of the step of cloning the nucleic acid of the cap gene(s) from such cell type of the animal.

A further embodiment of this invention is a method for the selection of a recombinant virion with a modified immunogenicity comprising the steps of
i) providing at least one first cell with a vector construct comprising at least one nucleic acid from the library of the invention together with a second nucleic acid (especially sequences such as AAV ITRs and one or more genes providing non-structural functions such as replication and packaging, for example functions of an AAV Rep protein) necessary for the packaging of virion.
ii) providing such first cell with necessary cellular, viral, physical and/or chemical helper functions for the packaging of virions if necessary;
iii) incubating such first cell under suitable conditions for the packaging of virions and collecting produced virions by such first-cell;
iv) applying an immunoselection step to the produced virions;
v) infecting at least one second cell with such collected virions;
vi) providing such second cell with necessary cellular, viral, physical and/or chemical helper functions for the packaging of a virion;
vii) incubating such second cell under suitable conditions for the packaging of virions and collecting produced virions by such first or second cell;
whereas steps iv) to vii) can be repeated several times.

The non-structural functions as for example the Rep protein can be provided in cis or in trans.

Furthermore, the first and the second cell can be of the same kind or type.

The immunoselection steps are well known in the art. One can think of various methods to use antibodies or similar molecules such as F_{AB} fragments or single chain antibodies to inhibit binding or uptake of virions by the second cell. For example one can pre-incubate the produced virions with monoclonal or polyclonal antibodies. If antibodies bind to a critical site of the virion that is involved in the mechanism of infection, this will result in a negative selection for virions recognized by such antibody. One could use either known monoclonal antibodies or sera from immunopositive mammals, especially humans. The polyclonal antibodies contained in this sera would have the advantage, that one can negatively select for virions that escape neutralizing antibodies without having the antibody isolated. A further immunoselection step of choice is an immunodepletion reaction using affinity chromatography with antibody columns. Column material such as CNBr-activated Sepharose can be used to bind monoclonal or polyclonal antibodies. Produced virions can then be incubated with such antibody column leading to an eluate of the column where binding virions have been depleted.

The invention further relates to a polypeptide comprising a peptide with the sequence RGDAVGV, RGDTPTS, GKLFVDR, RDNAVVP, GENQARS, RSNGVVP, RSNAVVP or NSVRAPP.

In a preferred embodiment, the polypeptide of the invention consists of a peptide with the sequence RGDAVGV, RGDTPTS, GKLFVDR, RDNAVVP, GENQARS, RSNGVVP, RSNAVVP or NSVRAPP.

According to a further preferred embodiment, the polypeptide of the invention is a Cap polypeptide, preferably derived from a parvovirus, especially from an AAV.

The invention further relates to the use of a polypeptide as defined above or comprising or consisting of a peptide with the sequence RGDXXXX, RGDXPXX, or DDDXPXX with the exception of AGTFALRGDNPQG, for the retargeting of eukaryotic viruses, preferably parvorviruses, especially AAV.

Furthermore, all identified peptides can be used for the targeting of non-viral vectors. Other potential uses of the peptides are triggering or blocking cellular pathways e.g. by the activation or inhibition of the receptor due to the binding of isolated peptides to the respective receptor. The peptides also can be used as fusions with other peptides or any other suitable molecules of choice. The peptides in this setting can be used to couple such fusion to the surface of a cell for the purpose of - for example - staining, tagging, sorting or killing of the cell.

The peptides can also be used for the purification of fusions or virions containing them by coupling the respective receptor onto beads and allowing binding of such fuions/virions to such coupled beads (affinity chromatography). Therefore such selected virions not only have the advantage of a changed cell specificity but also that they can be purified by affinity chromatography using their specific receptor.

The peptides RGDAVGV and RGDTPTS as well as RGDXXXX, RGDXPXX and DDDXPXX are useful in combination with cells that express RGD binding intergrins. RGD binding integrins are receptors that are widely expressed among eukaryotic cells (Ruoslahti E (1996) Annual Review of Cell and Developmental Biology 12, 697-715; Aumailley M et al. (1990): FEBS Lett 12:262(1):82-6). An example for an integrin that binds RGD motifs are the αᵥβ₅nd the αᵥβ₁ integrins. Such cells are for example megakaryocytes, e.g. the cell line used for the screening M-07e.

The peptides GKLFVDR, RDNAVVP, GENQARS, RSNGVVP, RSNAVVP or NSVRAPP are useful for B-CLL cells and Mecl cells. These peptides bind to one or more cellular receptors that have not been identified so far. Every cell or cell line that expresses one or more of these receptors is a potential target for these peptides. It is known in the art how to test a cell or cell line, if one of the peptides is capable of binding to the cell surface. Since these peptides were identified by screening the library against hematopoetic cells, it is reasonable to predict that many other hematopoetic cells will bind those peptides, for example B cells.

The invention further relates to a recombinant virion obtainable by the methods of the invention for the selection of a recombinant virion.

Furthermore, the invention relates to a mutant cap gene obtainable by the methods of the invention for the identification of a mutant cap gene.

Furthermore, the invention relates to a Cap protein encoded by the mutant cap gene of the invention.

Furthermore, the invention relates to a virion comprising the Cap protein of the invention.

Furthermore, the invention relates to a medicament for the treatment of a patient suffering from cancer, an autoimmune disesase, an infectious disease or a genetic defect comprising a virion, a cap gene or a Cap protein of the invention.

Furthermore, the invention relates to a method for treating a patient suffering from cancer, an autoimmune disesase, an infectious disease or a genetic defect comprising administering to the patient a virion, a cap gene, or a Cap protein of the invention.

In this document, the content of all cited documents is included by reference.

The following examples and figures are intended to explain the invention in detail without restricting it.

### Brief description of the Figures and Tables

**Fig. 1** **Schematic map of the plasmid pwt99oen.**

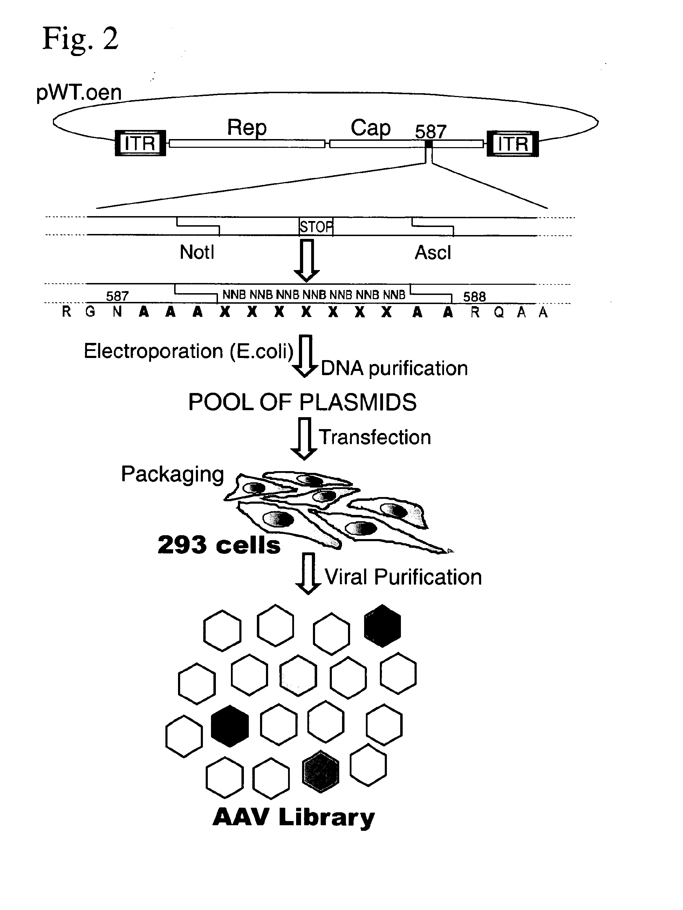

**Fig.2** **Construction of the library of AAV-2 capsid modified particles.** A pool of randomly generated oligonucleotides was cloned in an AAV-2 genome encoding plasmid at the site corresponding to amino acidic site 587 of capsid protein VP1. The obtained pool of plasmids was transfected into 293 cells. Following a standard virus production protocol, a library of approximately 10⁸ capsid modified AAV-2 clones was generated.

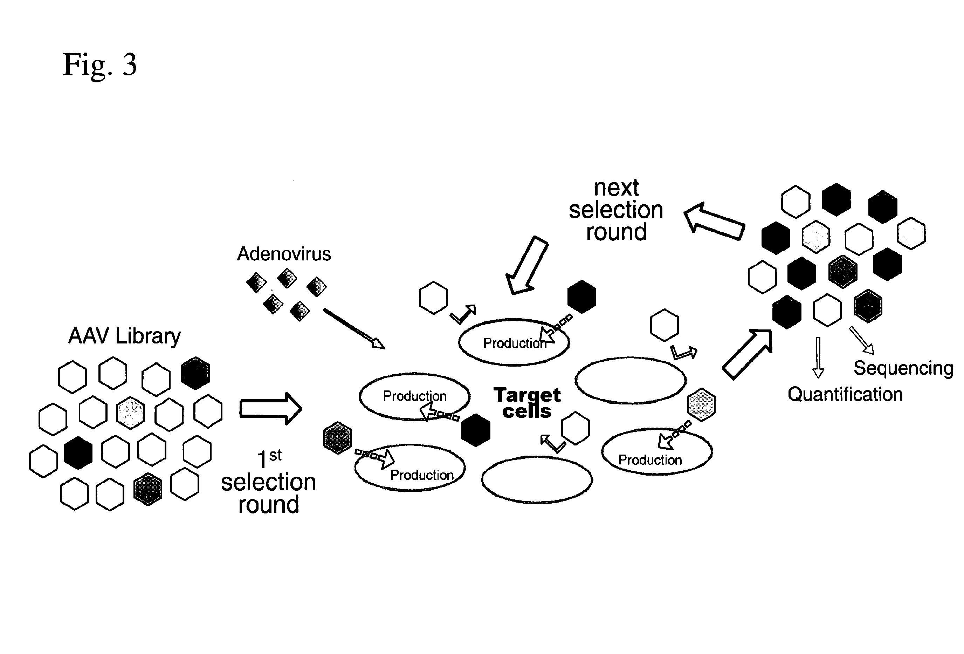

**Fig. 3** **AAV display screening procedure for the selection of retargeted mutants.** Target cells were infected with the library of capsid modified AAV-2 clones and with adenovirus (helper for AAV replication). Non infectious virions are removed by washing steps 2h post infection. The viral progeny collected 48h p.i. was used for the next selection round. The evolution of the AAV population after each round was monitored by titer determination and sequencing.

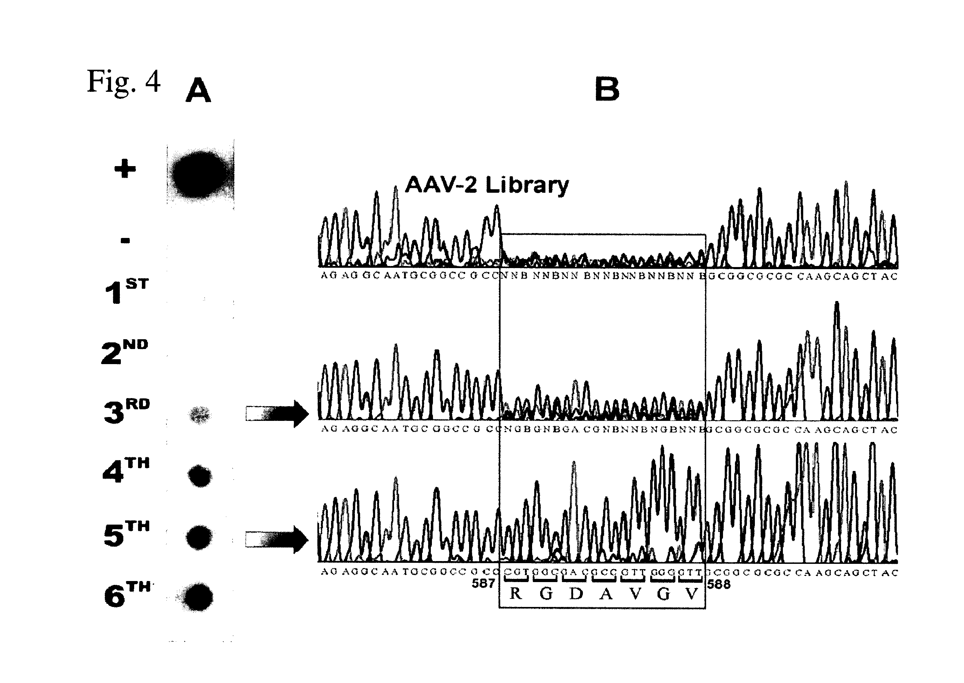

**Fig. 4** **Example of evolution of the viral population during 6 selection rounds on M07e cells.** (A) Dot blot assay quantification of viral progeny harvested after each infection cycle. (B) Sequencing of the random insertion containing region of the cap gene shows the progressive loss of heterogenity in the viral population collected after each selection round. After 5 rounds a single clone (in the shown example carrying a RGDAVGV inserted sequence) could be detected in the viral progeny.

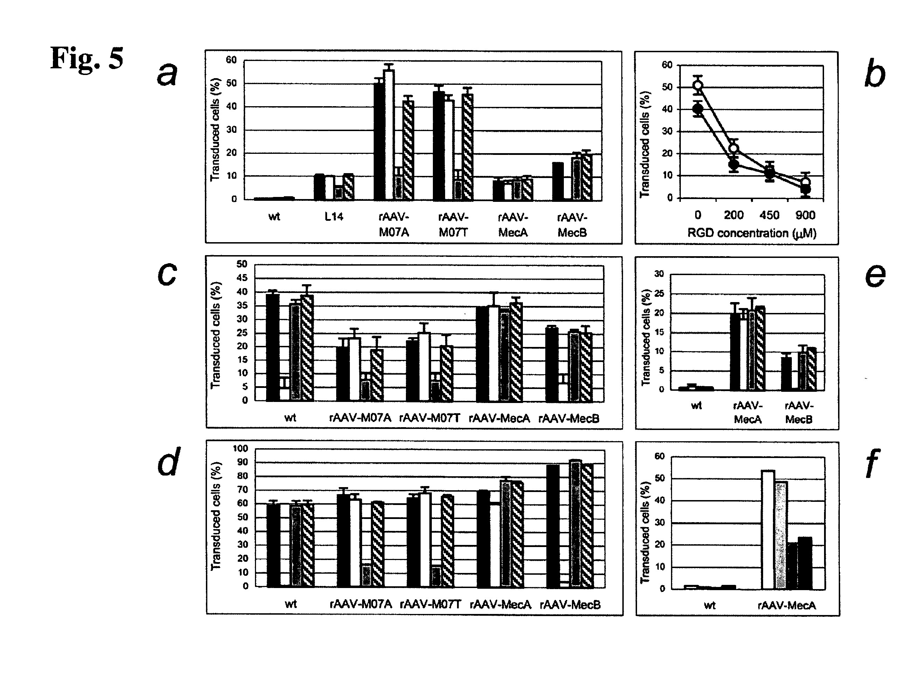

**Fig. 5** **Transduction efficiencies** Transduction efficiencies ± standard deviation as determined by FACS analysis in duplicate experiments for selected rAAV-GFP mutants (black bars). Transduction rates were also assessed after pre-incubation of viral preparation with soluble heparin (white bars) or pre-incubation of the cells with competing GRGDTP (gray bars) and inactive GRGES peptides (checked bars).
   a) M-07e cells.
   b) Concentration dependece of RGDTP mediated inhibition of M-07e cells transduction by rAAV/M07A (white circles) and rAAV-M07T (black circles).
   c) CO-115 cells.
   d) HeLa cells.
   e) Mec1 cells.
   f) Primary B-CLL cells obtained from four different patients.

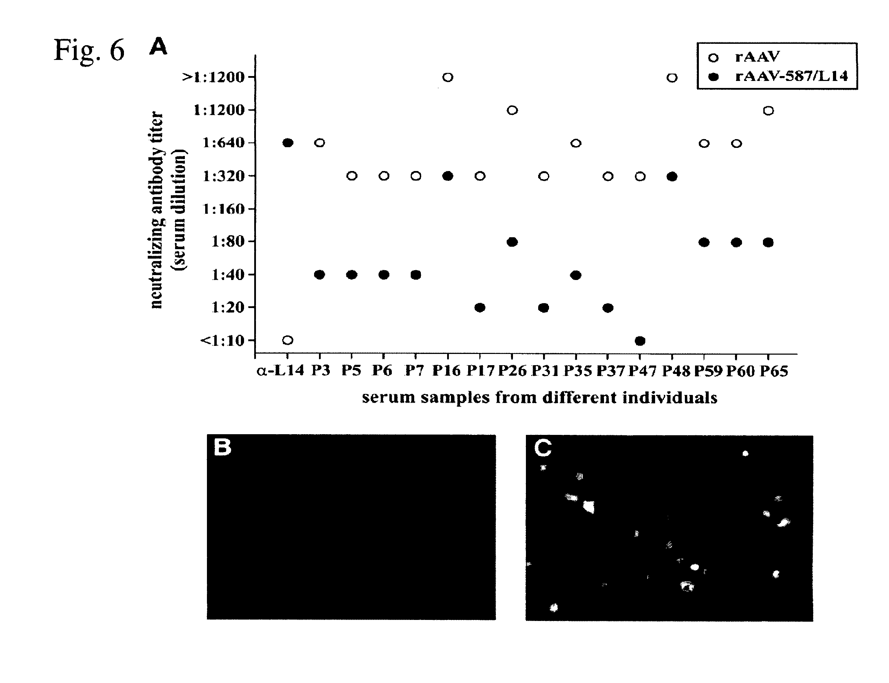

**Fig. 6**A neutralization assay on HeLa cells. (A) Neutralizing antibody titers against rAAV and rAAV-587/L14. Serial dilutions (1:10 - 1:1200) of 15 neutralizing human serum samples (P3 — P65) were analyzed on HeLa cells. As control, rabbit serum directed against the inserted L14-ligand (α-L14) was tested. The neutralizing titers (N₅₀) are expressed as the dilution at which transduction was 50% reduced compared to the positive control. rAAV (B) and rAAV-587/L14 (C) were incubated with serum P35 (1:80) prior infection of HeLa cells. GFP expression was monitored by fluorescence microscopy 48 hours post infection.

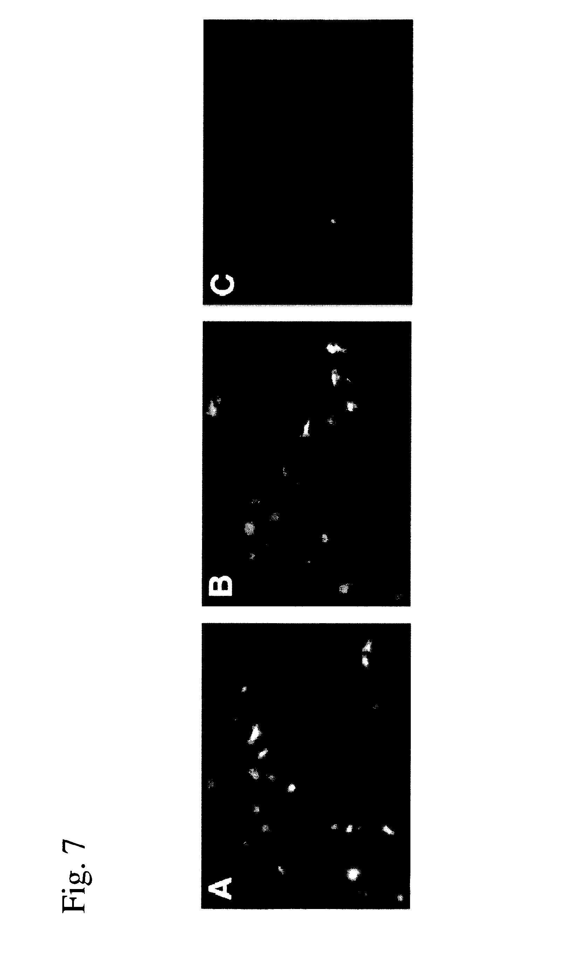

**Fig. 7**A neutralization assay on B16F10 cells. Infection of irradiated B16F10 cells with rAAV-587/L14 alone (A) or after co-incubation with P35 serum (C) or anti-L14 serum (D) at a 1:80 serum dilution. Cells were analyzed for GFP expression by fluorescence microscopy after 72 hours.

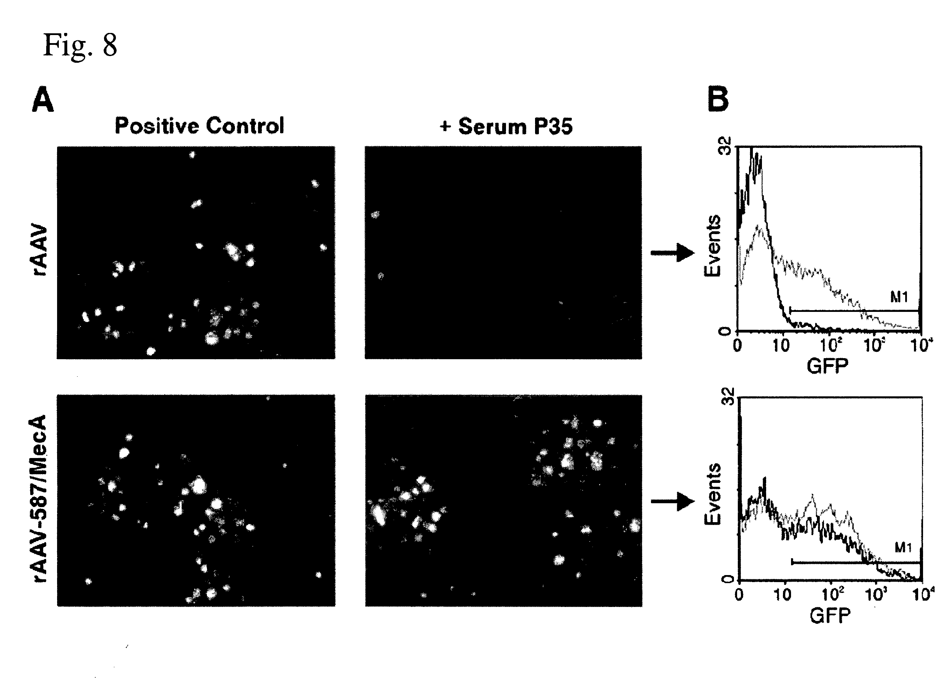

**Fig. 8** The effect of neutralizing antisera on rAAV-587/MecA transduction. (A) After infection with adenovirus, Mec1 cells were infected with rAAV (top row) and rAAV-587/MecA (bottom row) alone (positive control) or after co-incubation with serum P35 at a 1:80 dilution (+ serum P35). Note that more physical particles were used for rAAV to-achieve similar transduction: (B)-FACS analysis of rAAV (top row) and rAAV-587/MecA (bottom row) incubated with serum P35 (grey line) in comparison to their positive controls (black line). GFP expression was determined 48 hours post infection.
**Tab. 1 Characterization and specificity of rAAV-GFP mutants with capsid modifications**. Genomic titers were measured by dot blot assay. Infectivity of the mutants for Hela, M07e and Mec1 cells was measured by FACS analysis after infecting the cells with identical genomic particles/cell ratios. For each cell line, the transduction rate was normalized to 100% for the mutant corresponding to bold values. The ability of soluble heparin to inhibit infection of Hela cells was assessed preincubating viral preparations with soluble heparin.

### Examples

### Example 1: Methods

### Production of plasmids and viruses

For the construction of plasmid pWt.oen, the HCMV promoter/enhancer cassette and the GFP open reading frame in the plasmid pEGFPC-1 (Clontech, Palo Alto, California) were substituted with the wt AAV-2 genome encoding fragment of plasmid pUC-AV2 (Girod A et al (1999) supra). A DNA fragment encoding amino acids AAAstopA and the restriction sites Not1 and Asc1 was inserted between amino acid position 587 and 588 by PCR mutagenesis. To generate a library of AAV plasmids (p587Lib7) a pool of single strand DNA molecules was synthesized as 
5'-
TTGGCGCGCCGCVNNVNNVNNVNNVNNVNNVNNGGCGGCCGCTTTTT
TCCTTGA-3'
 (whereas N = A/G/C/T, V = A/G/C (not T))
and HPLC purified (Metabion GmbH, Martinsried, Germany). For the synthesis of double-stranded molecules a 5'-CTCAAGGAAAAAAGC-3' primer was used. dsDNA molecules were cloned into the Ascl-Notl large fragment of plasmid pWt.oen, p587Lib7 was electroporated into E. *coli* strain DH5α using a Gene Pulser (Biorad, Hercules, California) and amplified DNA was purified. The efficiency of the transformation was controlled by plating sample aliquots. DNA of more than 20 clones was controlled by sequencing with the primer 4066Back (5'-ATGTCCGTCCGTGTGTGG-3'). Plasmids pRC, pXX6 (obtained from J. Samulski, Chapel Hill, North Carolina) and psub/CEP4/EGFP were previously described (Girod A et al (1999) supra, Xiao X et al (1998) J. Virol. 72, 2224-32).

For the production of viruses, 15 150 mm Petri-dishes of 293 cells at 80% confluence were co-transfected with 37.5 µg of DNA. For the production of the AAV library, p587Lib7 and plasmid pXX6 were co-transfected at a molar ratio of 1:1. For the production of rAAV-wt, the cells were co-transfected with vector plasmid psub/CEP4/EGFP, packaging plasmid pRC and adenoviral plasmid pXX6 at a molar ratio of 1:1:1. For the production of the capsid modified GFP expressing rAAV mutants, pRC plasmids modified to contain the appropriate Notl-Ascl retargeting insertion were used. L14-AAV was produced using plasmid pI-587 instead of pRC (Girod A. et al supra). After 48 hrs cells were collected and pelleted by centrifugation. Cells were resuspended in 150 mM NaCI, 50 mM Tris-HCl (pH 8.5), freeze-thawed several times, and treated with Benzonase (50 U/ml) for 30 min at 37°C. Cell debris was removed by centrifugation, supernatant was loaded onto an iodixanol gradient and subjected to 69000 rpm for 1 hr at 18°C as described (Zolotukhin, S et al. (1999) Gene Ther. 6, 973-85). Virions were then harvested from the 40% iodixanol phase and titrated by DNA dot-blot hybridization (Girod A. et al., supra).

### Tissue culture

HeLa cells (human cervix epitheloid carcinoma, ATCC CCL 2), M-07e cells, a human megakaryocytic leukemia cell line (obtained from James D. Griffin, Boston, Massachussets), Mecl, a cell line derived from a patient with B-CLL in prolymphocytoid transformation (obtained from Federico Caligaris-Cappio, Torino, Italy), CO-115 cells (human colon carcinoma), and 293 cells (human embryonal kidney) were maintained in Dulbecco's modified Eagle's medium (DMEM) (HeLa and 293), DMEM/NUT.Mix.F-12 medium (CO-115), RPMI medium (M-07e) or Isocove's medium (Mecl) supplemented with 10% fetal calf serum (FCS), penicillin (100 U/ml) and streptomycin (I00 µg/ml), and L-glutamine (2 mM), at 37 °C and 5% CO₂. For M-07e cells, 10 ng/ml interleukin 3 (IL-3) was added to the medium.

Peripheral blood was obtained with informed consent from four patients with an established diagnosis of B-CLL. Mononuclear cells were isolated on a Ficoll/Hypaque (Seromed, Berlin, Germany) density gradient by centrifugation, depleted of monocytes by adherence to plastic tissue culture flasks and cultivated in Isocove's medium supplemented as for Mec1 cells. More than 98% of isolated-cells co-expressed CD5 and CD19 as assessed by flow cytometry, therefore non-malignant B cells did not constitute a meaningful fraction of the total cells isolated. Patients were either untreated or had not received cytoreductive treatment for a period of at least one month before investigation and were clinically stable and free from infectious complications.

### Determination of transduction efficiencies

Cells were seeded in 96 or 24 well plates (Nunc, Wiesbaden, Germany) and infected with rAAV-GFP clones, harvested 48 hrs p.i., washed and resuspended in 1 ml PBS. The percentage of GFP expressing cells was determined by flow cytometry with a Coulter Epics XL-MCL (Beckman Coulter, Krefeld, Germany). A minimum of 5000 cells were analyzed for each sample. Infectivity of the retargeted mutants was determined in the presence or absence of various concentrations of GRGDTP or GRGES peptides (Bachem, Bubendorf, Swiss) or 5 I.U./µI soluble heparin (Braun, Melsungen, Germany).

### Selection of AAV-2 retargeted mutants

10⁷ target cells were super-infected with 1000 genomic library particles/cell and with adenovirus at an MOI of 20 and incubated at 37°C. 2 hrs p.i. cells were centrifuged, resuspended in fresh culture medium and incubated at 37°C. 48 hrs p.i., cells were rinsed with 5 ml PBS, resuspended in 5 ml of lysis buffer (150 mM NaCI, 50 mM Tris/HCI, pH 8.5) and lysed through 3 freeze/thaw cycles. Cellular debris was removed by centrifugation and the supernatant was used to infect the next batch of target cells (second round of infection). After each selection round viral DNA was purified from a 100 µ1 aliquot of the crude lysates by phenol/chloroform extraction and the 587 region was sequenced (primer 4066-back).

### Example 2: Selection of AAV-2 retargeted mutants for M-07e and Mecl cells

We generated a-library of 4 x-10⁶ capsid -modified viral particles carrying random insertions of 7 amino acids at the position 587 (Fig. 2 and Example 1). The pool of capsid mutants was subjected to repeated cycles of infection and harvesting of the viral progeny from the target cells (Fig. 3). Virions with impaired ability to enter the cells were removed by changing the culture medium 2 hrs post infection (p.i.). Viral progeny was extracted from the cells 48 hrs p.i. by freeze/thaw cycles and used to infect a new batch of target cells in a new selection round. After each harvest, a small aliquot (100/tl) of the crude lysate was used to extract viral DNA. By titrating this DNA and sequencing the 587 region it was possible to monitor the evolution of the library (Fig. 4). The selective pressure provided by the culture environment drove the selection by means of their ability to accomplish every step in the infection process, namely binding, uptake, uncoating, nuclear translocation, replication and gene expression.

The potential of the AAV display system for the generation of retargeted mutants was tested on two cell lines that are resistant to wt AAV-2 infection.
M-07e is a human megakaryocytic cell line (Avanzi GC et al (1988) Br. J. Haematol. 69, 359-66). Failure of AAV-2 to infect these cells has justified the use of this cell line as negative control in several reported AAV-2 infection experiments Bartlett JS et al. (1999) Nat. Biotechnol. 17, 181-186; Ponnazhagan S et al (1996) J. Gen. Virol. 77, 1111-22).
Mecl is a cell line derived from B-cell chronic lymphocytic leukemia (B-CLL) cells in prolymphoid transformation (Stacchini A et al. (1999) Leuk. Res. 23, 127-36) and is also resistant to wt AAV-2 infection.

A typical selection is depicted in Fig. 4. The amount of viral DNA detected in the crude lysates and the analysis of the sequence showed that the number of recovered virions increased after each round, while the heterogeneity of the pool was progressively lost. After 5 rounds only one single clone was present in the viral progeny. Application of the library to M-07e cells led to the selection of a clone carrying an RGDAVGV sequence at the 587 site (Fig. 4). In a parallel experiment we isolated a clone which carried an RGDTPTS sequence. Interestingly, both clones isolated from M-07e cells led to the selection of an RGD motif, known to bind to several types of cellular integrins (Ruoslahti E (1996) Annu. Rev. Cell. Dev. Biol 12, 697-715). Analogous experiments performed with Mecl cells led to the identification of clones carrying GENQARS, GKLFVDR, NSVRAPP and RSNAVVP/RSNGVVP peptides, respectively (data not shown).

### Example 3: Cloning of selected mutants

The selected DNA sequences were cloned into appropriate plasmids for the production of capsid-modified recombinant AAV (rAAV) vectors encoding the enhanced Green Fluorescent Protein (GFP). Corresponding GFP-expressing retargeted vectors rAAV-M07A (RGDAVGV insertion), rAAV-M07T (RGDTPTS insertion), rAAV-MecA (GENQARS insertion) and rAAV-MecB (RSNAVVP insertion) were produced (see Example 1) and genomic titers were determined by dot blot assay. Genomic titers of the selected mutants were comparable or higher than titers of AAV vectors with unmodified capsid (rAAV-wt) (Tab. 1).

### Example 4: Transduction efficiencies of retargeted vectors

The selected capsid mutants were tested for their ability to transduce M-07e cells (Fig. 5a). At a genomic particle/cell ratio of 2x10⁴, the mutants rAAV-M07A and rAAV-M07T transduced 50 ± 2.5% and 47 ± 2.7% of M-07e cells, respectively, representing a 100 and 94 fold increase in comparison to rAAV-wt transduction efficiency (0.5 ± 0.01%). In contrast, rAAV-MecA and rAAV-MecB transduced M-07e cells with an efficiency of only 8.1 ± 1.5% and 16 ± 2%. The vector rAAV-L14, carrying an RGD motif inserted at position 587 (Girod A et al (1999) supra), was also compared. Interestingly, rAAV-L₁4 transduced only 10 ± 0.7% of M-07e cells, which was five times less efficient than the selected mutants rAAV-M07A and rAAV-M07T. This highlighted the advantage of the combinatorial approach when compared with the simple insertion of an exogenous sequence.

### Example 5: Tropism of retargeted vectors

We then examined whether the transduction of M-07e cells by rAAV-M07A and rAAV-M07T vectors was specifically mediated by the amino acids inserted at position 587. In the capsid of wt AAV, the region around position 587 is involved in the binding to heparan sulfate proteoglycan (HSPG) (Nicklin SA et al. (2001) Mol. Ther. 4, 174-81; Wu P. et al. (2000) J. Virol. 74, 8635-47), the primary receptor of AAV-2 (Summerford C and Samulski J (1998) J. Virol. 72, 1438-45). Pre-incubation with soluble heparin, an HSPG analogue and competitor, inhibited transduction of M-07e cells by rAAV-MecB but not by rAAV-M07A, rAAV-M07T and rAAV-MecA (Fig. 5a). This indicated that the insertion of appropriate heterologous amino acids at this site abolished the requirement of AAV to use HSPG as a primary receptor for transmembrane entry. In marked contrast, preincubation of M-07e cells with a competing soluble GRGDTP peptide (450 µM) almost completely inhibited transduction of M-07e cells by rAAV-M07A and rAAV-M07T (Fig. 5a). This effect was concentration-dependent (Fig. 5b). Preincubation with an inactive (GRGES) peptide (450 µM) had no effect (Fig. 5a). Taken together, the results demonstrate that rAAV-M07A and rAAV-M07T transduce target cells through the specific interaction of the selected RGD motif presented on the viral capsid with an integrin receptor expressed on the surface of the target cells.

We also examined the selected mutants on cells which expressed HSPG and were permissive for wt AAV-2 infection. In human colon carcinoma CO-115 cells (Carrel S et al. (1976) Cancer Res. 36, 3978-84) the transduction efficiency of the virus mutants rAA-V-M07A, - rAAV=M07T; rAAV-MecA - and rAAV=MecB was reduced by 50, 43, 12 and 31%, respectively, when compared to wt AAV-2 (Fig. 5c), while it was similar to wt AAV-2 in HeLa cells (Fig. 5d). In both cell lines, transduction by mutants rAAV-M07A and rAAV-M07T was blocked almost completely by the GRGDTP peptide, but not by the GRGES peptide nor by heparin. In contrast, transduction by rAAV-wt and rAAV-MecB was inhibited by heparin but not by the GRGDTP peptide (Fig. 5c and d). Moreover, cells which lacked the expression of an integrin receptor were not permissive for transduction by the mutants rAAV-M07A and rAAV-M07T (data not shown). Taken together, these results demonstrate that the integrin receptor recognizing the RGD peptide on rAAV-M07A and rAAV-M07T capsids is also expressed on CO-115 and HeLa cells. Therefore, the tropism of the selected capsid mutants is not restricted to hematopoietic cell lines, but to an integrin receptor, which is probably widely expressed.

Successful retargeting of mutants selected on Mecl cells is depicted in Fig. 5e. While transduction of Mecl cells by rAAV-wt was not detectable, mutants rAAV-MecA and rAAV-MecB transduced up to 23% of these cells at a genomic particle/cell ratio of 4x10⁴. Using rAAV-MecA, we then examined the transduction efficiency in primary leukemia cells in order to explore the potential clinical relevance of the AAV display technology. Primary B-CLL cells are resistant to transduction by most currently available vital vector systems, including AAV (Cantwell MJ et al (1996) Blood 88, 4676-83; Rohr UP et al (1999) Blood 94, 181a). In remarkable contrast to vectors with unmodified capsid, rAAV-MecA (8x10⁴ genomic particles/cell) transduced primary leukemia cells isolated from four B-CLL patients at an efficiency of 54, 49, 23 and 21%, respectively (Fig. 5f). In contrast, rAAV-M07A and rAAV-M07T failed to transduce primary B-CLL cells (data not shown). The results indicate that such modified vectors might be useful for an AAV-based gene therapy of B-CLL Cantwell M et al. (1997) Nature Med. 3, 984-89; Wierda WG et al. (2000) Blood 96, 2917-24).

Any successful attempt to molecularly engineer viruses for human somatic gene therapy will depend on our ability to generate retargeting vectors that retain the major functions required for appropriate intracellular processing. Our findings seem highly relevant in this regard. Because of the complexity of the virus-cell interaction, it is highly advantageous to screen appropriate virus mutants from a large library rather than to generate a limited number of virus variants by a more or less educated guess. Since no refinement of the selection process was undertaken, some limitations remained: the capsid mutants showed receptor specificity, but not cell specificity. However, the goal of producing viral clones with a further restriction of the virus tropism should be achieved by adding steps to the screening process which deplete those clones able to infect undesirable cell types. An additional upgrade of this technology might be the generation of an AAV library with randomized insertions in multiple sites of the capsid. Moreover, the virus display might be also used for the identification of capsid variants that are less efficiently recognized by human antibodies or immune effector cells. Finally, the shortness of the insertions that were successfully used to generate retargeting clones suggests that this technology might be applicable in other viral systems.

### Example 6

In two completely independent experiments, after 5 selection rounds on M07e cells (resistant to wt AAV-2 infection) the sequences obtained showed no more randomized features at the site of the insertion and we were able to characterize two highly homologue RGD motifs containing sequences: RGDAVGV and RGDTPTS.

Oligonucleotides encoding for these peptidic sequences were cloned into GFP-AAV plasmids. The correspondent mutants were packaged and used to infect MO7e cells. For both mutants, infection of M07e cells with 2000 genomic particles/cell resulted in transduction rates higher than 86% (wt AAV-2 transduction rate was less than 6%).

Selection rounds performed on a cell line (Mecl) derived from B-CLL cells in prolymphoid transformation, led to the identification of several sequences that provided AAV capsids with improved infection efficiency on these cell types. In particular the sequence GENQARS conferred to GFP-AAV virions transduction rates of up to 20% on Mecl cells, and to 55% on primary B-CLL cells (both cell types are non-permissive to wt AAV infection).

### Production of the AAV library.

The cloning strategy is depicted in Fig. 2. A combinatorial library of AAV for the selection of retargeted clones was generated by cloning randomly generated oligonucleotides with a length of 21 bases at the genomic site corresponding to aa position 587 using the plasmid pWT99oen (Fig. 1, sequence given).

The inserted sequence consisted of 7 repetitions of NNB codons (N=A,C,G, or T; B=C,G or T) to allow a 50% reduction of stop codons probability.

While the wild type aminoacids flanking the 587 position were all retained, a triple and a double Ala sequence was engineered upstream and downstream of the randomized sequence, respectively, in order to increase the flexibility of the inserted peptide and to reduce conformational stress of the native capsid structure.

We obtained approximately 5x10⁷ plasmids containing the randomly generated insertions. More than 20 of these plasmids were sequenced to control the outcome of the cloning; all the sequenced plasmids contained a randomized 21 bases insertion in the correct position.

This pool of plasmids was transfected into 293 packaging cells concomitantly to a helper plasmid containing the genes of adenovirus, necessary for the packaging of AAV virions.

Viral progeny was harvested by a standard purification protocol on an iodixanol discontinuous gradient (Samulski et al.).

Genomic and infectious titers of the viral preparation were measured by dot blot and immunofluorescence analysis using an anti rep antibody and quantified in respectively 4x10¹¹ virions/ml and 6x10⁸/ml.

The sequence obtained after digestion of viral proteins with ProteinaseK and phenol/chloroform extraction is depicted in Fig. 4 and confirms the randomized nature of the insertion at the 587 site.

### Selection of Efficiently Infecting Mutants on target Cells.

To demonstrate the feasibility of the combinatorial selection approach, we performed several infection and harvest rounds of the AAV library on Mo7e and Mec1 cells (both cell lines being almost completely resistant to wild type AAV infection) in order to isolate the clones with better infection ability. This was simply achieved by performing repeated infection/harvesting cycles on the target cells. A schematic representation of the procedure is depicted in Fig. 3. Adenovirus at a MOI of 100 was used as helper for the replication of AAV.

In this system, the cultural environment exerts a strong selective pressure contemporarily on binding, entry, replication and packaging ability of the viral clones. Viral replication itself exerts in the infected cells the amplification step necessary to augment the number of viable mutants that will be harvested and used for the subsequent selection rounds.

2 hours p.i., the culture medium was changed to remove non-infectious mutants. 48 hours p.i. cells were centrifuged, rinsed with PBS, resuspended in 5 ml lysis buffer and subjected to 3 freeze/thaw cycles to allow diffusion of the progeny virions into the solution. Cellular debris was separated by centrifugation at 5000 g.

After each infection/harvesting round, a small aliquot of the crude lysate preparation was used to measure the genomic titer of the preparation and for sequencing of the respective viral population. The remaining preparation was used to infect the next batch of target cells.

### Identification and characterization of mutants retargeted to M07e cells

The M07e cell line is resistant to wt AAV-2 infection. This characteristic has been attributed to the lack of expression of the putative primary receptor for AAV-2 (heparan sulfate proteoglycan), and has justified the use of this cell line as negative control in many reported AAV-2 infection experiments.

Fig. 4 shows the results of 5 rounds of infection/harvesting of the AAV pool on this target cells. Round after round, we could observe a slight increase of the AAV genomic titer in the crude lysates preparation (as assessed by dot blot analysis). Concomitantly, the peaks of the sequence-reaction chart in the random insertion portion became increasingly higher during the selection procedure, and at the 5^{th} cycle it was possible to read a fixed sequence from the sequencing reaction.

The selection procedure was performed in two independent experiments and resulted in the identification of two DNA sequences encoding for respectively RGDAVGV and RGDTPTS peptides. Figure 4 depicts only the experiment that generated the RGDAVGV sequence.

The selected DNA sequences were cloned into appropriate plasmids for the production of capsid-modified recombinant AAV vectors encoding for the Enhanced Green Fluoresent Protein (rAAV-GFP). GFP expressing versions of these retargeted clones (rAAV-M07A containing the RGDAVGV sequence and rAAV-M07T containing the RGDTPTS sequence) were produced by standard rAAV production protocols.

The ability of mutants rAAV-M07A and rAAV-M07T to transduce M07e cells was compared with the efficiencies of vectors with unmodified capsid (rAAV-wt) and of vectors expressing the L14 sequence at the 587 site (rAAV-L14). M07e cells were infected with identical genomic particles/cell ratios (Fig. 5). Transduction rates were higher than 88% when using the retargeted mutants, 6% using unmodified capsid mutants and 18% using rAAV-L14.

Similarly to the selected mutants, rAAV-L14 carries a RGD motif containing sequence (of the laminin fragment P1) inserted at the 587 site. The more than 5 fold higher efficiency of the mutants generated by our display system in comparison with rAAV-L14 clearly highlights the advantages of the combinatorial display approach where the modifications are selected for their efficiency directly in the vector contest, in comparison with the simple insertion of a previously known retargeting sequence.

To demonstrate the specificity and the receptor-mediated nature of the infection process, we measured M07e cell transduction rates of the viral clones in the presence of a competing RGDS peptide or an inactive RGES peptide. Incubation of target cells with 100uM RGDS peptides prior to infection reduced transduction efficiencies by more than 50%. Pre-incubation of the cells with the RGES peptide failed to inhibit infection (Fig. 5A):

### Identification and characterization of B-CLL cells retargeted mutants.

Mecl is a cell line derived from B-Cell Chronic Lymphocytic Leukemia cells in prolymphoid transformation (Stacchini et al.) and is resistant to wt AAV-2 infection.

After 3 rounds of selection on Mecl cells, it was possible to read an inserted GANGANNACNNNNCNANNANN nucleotidic sequence at the 587 site.

In other setups of the selection procedure on this type of cells, after 5 rounds we could isolate viral clones with insertions encoding for peptides GKLFVDR, GENQARS, RSNGWP, or NSVRAPP.

The GENQARS sequence was cloned into an appropriate plasmid for the production of capsid-modified recombinant AAV vectors encoding for the Enhanced Green Fluoresent Protein (rAAV-GFP). GFP expressing viral particles of this retargeted clone (rAAV-Mecl) were produced by standard rAAV production protocols.

Infection of Mecl cells with rAAV-Mecl (20000 genomic particles/cell) resulted in a transduction rate of approximately 20%, while rAAV-wt failed to transduce more than 2% of these cells (Fig. 5 B).

Primary B-CLL cells are resistant to transduction with most currently available viral vector systems and previous reports failed to show for AAV vectors transduction rates greater than 3% (citation from David's paper). rAAV-Mecl showed transduction efficiencies of 54, 49, 21 and 23% when applied to primary cells obtained from 4 B-CLL patients (Fig. 5 C). With-these transduction rates, an AAV-based gene therapy approach for the cure of B-cell Chronic Lymphocytic Leukemia is now possible for the first time. Moreover, patient-specific differences in the permissivity of these primary cells to AAV vectors are suggested by these results and confirmed by other data obtained (Wendtner et al. paper submitted). The virus-display technology opens the horizon for the generation of patient specific vectors. Optimization of the protocol for the selection of retargeted mutants directly on primary B-CLL cells shall lead to achieve this goal and efforts in this direction are currently spent in our laboratory.

### Example 7

### Transduction of HeLa cells by rAAV-587/L14 is not inhibited by preexisting neutralizing antibodies in human serum samples

A detailed understanding of major immunogenic domains on the adeno-associated virus (AAV) capsid is not only important with regard to the binding of serum antibodies to the virus and its subsequent neutralization by the immune system, but also with regard to the existence of neutralizing antibodies that directly inhibit infection of the target cells by AAV vectors. To analyze the interference of different human antisera with AAV transduction, we used a recombinant AAV vector coding for GFP and carrying the L14 ligand at position 587 (rAAV-587/L14) to determine whether this modification would block the neutralizing ability of human antisera.

First, we determined the presence of neutralizing antibodies in human serum samples. 43 serum samples positive for AAV antibodies (Ab) were tested in a neutralization assay with an AAV vector coding for GFP, which carried the wild-type AAV capsid (rAAV). rAAV was incubated with serial dilutions of serum samples prior to transduction of HeLa cells. Thereafter, the number of GFP expressing cells was assessed by FACS analysis. Neutralizing titers were defined as the serum dilution where transduction was-reduced by 50% (N₅₀): Serum samples were considered as neutralizing when the N₅₀ was 1:320 or higher. 31 of these 43 serum samples (72%) contained neutralizing Ab against AAV, in agreement with previously published data (Erles K et al. (1999) J Med Virol 59: 406-11).

15 of these 31 serum samples were randomly selected for further analysis. The effect of these serum samples on the transduction of HeLa cells by rAAV-587/L14 as compared with rAAV was determined (Fig. 6A). In addition, the neutralizing monoclonal Ab C37-B (Wobus CE et al. (2000) J Virol 74: 9281-93) and an anti-L14 serum (generated against the L14 ligand) were tested. For these experiments identical transducing particle numbers of rAAV-587/L14 and rAAV were used. Both vectors were incubated with serial dilutions of neutralizing serum samples prior to transduction of HeLa cells. For all serum samples tested, transduction by rAAV-587/L14 was 8 up to 64 fold less reduced than transduction by rAAV (mean 15 fold). In 13 out of 15 serum samples, transduction by rAAV-587/L14 was only slightly impaired, with neutralizing titers of 1:80 or lower, demonstrating the ability of rAAV-587/L14 to escape the effects of neutralizing Ab (Fig. 6A). Strikingly, rAAV-587/L14 was able to escape the neutralizing Ab in serum P47 at any dilution tested, and serum samples P17, P31 and P37 reduced transduction only at a dilution of 1:20, where unspecific interactions could not be excluded. Figures 6B and 6C show one representative experiment with serum P35, which completely inhibited transduction by rAAV at a 1:80 dilution (fig. 6B). In marked contrast, transduction by rAAV-587/L14 was not affected (Fig. 6C). Only two serum samples (P16 and P48) were able to neutralize rAAV-587/L14 transduction efficiently, with a N₅₀ of 1:320. We assume that this was due to the high neutralizing Ab content in these serum samples, because transduction by rAAV-587/L14 still remained less affected than transduction by rAAV. As an additional control, the monoclonal Ab C37-B was tested. C37-B is a neutralizing Ab that inhibits binding of AAV to the host cell (Wobus CE et al. supra). It failed to bind rAAV-587/L14 in an ELISA (data not shown), therefore it should not interfere with rAAV-587/L14 transduction. As expected, rAAV-587/L14 transduction was not neutralize by G37-B, while-rAAV transduction could-be totally inhibited by this antibody (data not shown). In marked contrast, anti-L14 serum, which was generated against the L14 ligand, neutralized rAAV-587/L14 transduction completely at a 1:160 dilution, while rAAV transduction remained unaffected (Fig. 6A). To rule out the possibility that these observations were due to different numbers of physical particles used for rAAV and rAAV-587/L14, we performed additional control experiments, where neutralization assays were performed with identical numbers of physical particles for both AAV vectors. These experiments yielded identical results (data not shown).

Taken together, these results demonstrate that the mutant rAAV-587/L14 is able to escape preexisting neutralizing Ab in human serum samples.

### Neutralizing sera do not interfere with the L14 mediated tropism of rAAV-587/L14 on B16F10 cells

Insertion of the integrin specific L14 peptide in 587 expands the tropism of AAV to non-permissive B16F10 cells (Girod A et al. (1999) Nat Med 5: 1052-6). To determine if rAAV-587/L14 was able to retain its ability to infect the target cell line B16F10 via the inserted ligand L14 in the presence of neutralizing antisera, we performed additional experiments with selected serum samples. rAAV-587/L14 was incubated with serial dilutions of P35 serum before transduction of irradiated B16F10 cells. After 72 hours GFP expression was measured, rAAV-587/L14 efficiently transduced B16F10 cells despite incubation with P35 at a 1:80 dilution, whereas anti-L14 serum completely inhibited transduction at this dilution (Fig. 7B and 7C). When testing P37 and P26, the same neutralizing titers as determined on HeLa cells were obtained (data not shown). These findings showed that the AAV L14 targeting vector could escape neutralizing antibodies in human sera while retaining its retargeting ability.

### The ability of rAAV-587 to escape neutralizing sera does not depend on the inserted L14 ligand

To exclude that the escape from neutralizing antisera was caused by a specific ligand, we tested another insertion mutant, rAAV-587/MecA that carries a 7 aa ligand (GENQARS) at position 587. This mutant has been selected by AAV-display on Mecl cells and efficiently transduces Mecl cells and primary B-cells from chronic lymphocytic leukemia patients in a receptor specific manner (as described before). rAAV-587/MecA and rAAV were incubated with the serum P35 before Mecl cells were infected. Transduction of Mecl cells by rAAV-587/MecA was not affected by the neutralizing Ab of serum P35 (1:80 dilution). In contrast, rAAV transduction was almost completely inhibited by this serum (Fig. 8). Experiments with other neutralizing serum samples provided identical results (data not shown). As with HeLa cells, A20 was able to inhibit transduction of rAAV-587/MecA, while C37-B had no effect (data not shown).

Taken together, the results demonstrate that the insertion of different heterologous ligands at position 587 allows escape from preexisting neutralizing antibodies. Targeting properties of these vectors are retained in these capsid mutants, even in the presence of neutralizing antisera.

### Discussion

This is the first description of the generation and application of an eukaryotic virus combinatorial library for the identification of cell type specific viral gene delivery vectors. In the described experiments, the application of this techniques resulted in the description of several new AAV mutants with high efficiency of transduction of cells that are resistant to wt AAV infection.

All mutants described herein are specific, and show tropism characteristics determined by the insertion at the 587 site, as demonstrated by the RGDS peptide competition experiments (Fig. 5 A). Also these clones showed no interaction with the natural primary receptor of AAV, heparan sulfate proteoglycan (Tab.1). Further specificity of the virions could be achieved introducing other modifications of the capsid structure, e.g. combining the insertion of retargeting sequences with modifications such as 561-565 DEEEI-AAAAI substitution (Wu et al.), and/or the AISP insertion at nucleotidic site 3761 (Rabinowitz et al.).

Another possibility to increase specificity of the vectors is the introduction of subtractive selection rounds, e.g. infecting cells for which the infection is undesired and recovering the non-infectious virions containing supernatant, or using affinity columns to deplete the viral population from column-binding clones.

A further upgrade of the system that is underway in our lab is the generation of an AAV library with randomized insertions at the level of multiple capsid protein sites.

The description of the mutant GENQARS, that showed transduction rates up to 55% of B-CLL primary cells, has immediate relevance for gene therapy of this malignancy. Previous attempts to transduce these cell type with AAV-based vectors failed to achieve efficiencies greater than 3%. Our results also suggest the importance of establishing protocols for the generation of patient-specific vectors. With the AAV Display technology this is now possible.

The comparison of the efficiency of infection of the randomly selected mutants with the L14 mutant, demonstrates the importance of the specific position of the RGD sequence, and of the flanking amino acids and therefore suggests the advantages of selecting the retargeting modifications directly in the structural context of the vector.

The technology described herein for the adeno-associated virus can be adapted for any viral system.

Besides the goals of gene delivery systems, the AAV display will be a valuable tool for the understanding of the biology of this virus, for the characterization of peptides with interesting biological properties, and for the investigation of specific ligand-receptor interactions.

**TAB. 1**

| **Viral Clone** | **Genomic Titer/ml** | **Infectivity on Hela** | **Infectivity on M07e** | **Infectivity on B-CLL cells** | **Heparin Inhibition** |
|---|---|---|---|---|---|
| **wt** | 5 ×10¹⁰ | **100%** | 1% | 3% | + |
| **L14** | 10¹⁰ | 2% | 20% | n.d. | - |
| **rAAV-M07A** | 10¹¹ | 108% | **100%** | 10% | - |
| **rAAV-M07T** | 10¹¹ | 91% | **84%** | 29% | - |
| **rAAV-Mec1** | 5 × 10¹⁰ | 100% | 39% | **100%** | - |

The following embodiments are especially preferred:
1. A method for the production of a library of nucleic acids comprising a multiplicity of expressible structural genes from at least one eukaryotic virus, comprising the steps of:
   a) providing a set of nucleic acids, each encoding at least one structural gene from a eukaryotic virus and comprising a suitable packaging sequence, and
   b) inserting a first insert (1) into the structural gene.
2. The method of 1 to 3, wherein the structural genes are from an enveloped virus such as a retrovirus, lentivirus, herpes virus, e.g. HSV1, HSV2, EBV, Varizella zoster virus, human herpes virus 1, 2, 3, 4, 7 or 8.
3. The method of 1, wherein the structural genes are cap genes from a non-enveloped virus such as parvovirus or adenovirus.
4. The method of 3, wherein the cap genes are from a parvovirus selected from the group consisting of Adeno-associated Virus (AAV), Canine Parvovirus (CPV), MVM, B19, H1, AAAV or GPV.
5. The method of 4, wherein the cap genes are from an AAV.
6. The method of 1 to 5, wherein the set of nucleic acids is derived from one nucleic acid.
7. The method according to 1 to 6, wherein by inserting insert (1) a sequence of the structural gene is removed.
8. The method according to 7, wherein the removed sequence comprises or is part of an insert (2) inserted into the structural gene before step (a).
9. The method according to 8, wherein insert (2) prevents the formation of a functional capsid protein, preferably by containing a stop codon.
10. The method according to 9, wherein by inserting insert (1) the stop codon is removed.
11. The method according to 1 to 10, wherein the number of nucleotides of insert (1) and/or insert (2), preferably of insert (1) and insert (2), is three or a multiple of three.
12. The method of 1 to 12, wherein insert (1) is inserted at a region of the cap gene encoding amino acids on the surface of the structural protein.
13. The method of 12, wherein the virus is AAV and wherein the insert (1) is inserted after a nucleic acid corresponding to any site within the first amino terminal amino acids 1 to 50, or corresponding to amino acid positions 261, 381, 447, 534, 573, and/or 587 of the capsid protein VP1, preferably to amino acid position 447 or 587.
14. The method of 1 to 13, wherein insert (1) is randomly or partially randomly generated.
15. The method of 1 to 14, wherein insert (1) does not contain any stop codons.
16. The method of 1 to 15, wherein the library has a multiplicity of viral mutants that is greater than 10², preferably greater than 10⁵, especially greater than 10⁶.
17. A library of nucleic acids comprising a multiplicity of expressible structural genes, preferably cap genes from a parvovirus, more preferably from an Adeno-associated Virus (AAV), an Canine Parvovirus (CPV), MVM, B19, H1, AAAV or GPV.
18. The library according to 17, wherein the multiplicity of expressible structural genes, preferably cap genes is greater than 10², preferably greater that 10⁵, especially greater that 10⁶.
19. A library according to 17 or 18, wherein the library has a multiplicity of viral mutants that is greater than 10², preferably greater than 10⁵, especially greater than 10⁶.
20. The library according to 17 to 19, wherein the nucleic acid is a linear nucleic acid, a plasmid, a viral particle or a viral vector, e.g. a recombinant AAV, Adenovirus or Herpes Simplex Virus vector.
21. The library according to any of the 17 to 20, wherein the nucleic acid further comprises packaging sequences such as AAV ITRs and at least one expressible gene providing necessary functions for replication and packaging of virions such as an AAV Rep protein.
22. The library according to any of the 17 to 21, wherein the nucleic acid is DNA.
23. The library according to any of the 17 to 22, wherein the cap gene is derived from one of the AAV serotypes from the group comprising AAV1, AAV2, AAV3, AAV4, AAV5 and AAV6.
24. The library according to any of 17 to 23 wherein the cap gene is derived from the cap gene encoded in plasmid pWT99oen.
25. The library according to any of 17 to 24, wherein a multiplicity of nucleic acid sequences are-inserted into at least one site of the structural gene, preferably of the cap gene, wherein the number of inserted nucleotides is three or a multiple of three.
26. The library according to 25, wherein the inserted nucleic acid sequences are randomly generated, especially using NNN codons, NNB codons or NNK codons.
27. The library according to 25, wherein the inserted nucleic acid sequences are partially randomly generated, especially using codons with one, two or three fixed nucleotides.
28. The library according to any of 25 to 27, wherein the inserted nucleic acid sequences have a length of at least 3 nucleotides, preferably of at least 9, especially at least 18 nucleotides.
29. The library according to any of the 25 to 28, wherein the inserted nucleic acid sequences were inserted using standard restriction endonucleases or recombination systems, preferably the gateway or the cre/lox recombination system or polymerase chain reaction techniques, preferably using degenerated primers.
30. The library according to any of the 25 to 29, wherein the inserted nucleic acid sequences lead to an insertion of amino acids into the VP1, VP2 and/or VP3 structural protein, preferably at a site that is located on the surface of the capsid.
31. The library according to any of the 25 to 30, wherein the inserted nucleic acid sequences are inserted after a nucleic acid corresponding to any site within the first amino terminal amino acids 1 to 50 of VP 1, or corresponding to amino acid positions 261, 381, 447, 534, 573, and/or 587 of VP1, preferably to amino acid position 447 or 587.
32. The library according to any of 17 to 31, wherein the structural gene, preferably the cap gene has at least one further mutation leading to for example at least one point mutation, at least one internal deletion, insertion and/or substitution of one or several amino acids or at least one Nor C-terminal deletion, insertion and/or substitution of one or several amino acids, or a combination of these mutations, preferably a mutation inhibiting heparansulfate proteoglycan binding, integrin and/or Fibroblast Growth Factor Receptor (FGFR) binding.
33. The library according to any of 17 to 32, wherein the structural gene, preferably the cap gene has a further constant insertion of at least one codon upstream and/or downstream of the insertion site of the inserted nucleic acid sequence, preferably of one or two or three codons coding for Ala, Gly, Leu, Ile, Asp and/or Arg, especially an insertion of three Ala upstream and two Ala downstream of the insertion site.
34. A library of nucleic acids comprising a multiplicity of expressible structural genes from at least one eukaryotic virus, obtainable by the method of any of 1 to 16.
35. The library according to 34, having the features as defined in 17 to 33.
36. A library of virions, especially parvovirus virions, with capsid protein modifications.
37. The library of virions according to 36, containing particles containing the genetic information necessary to generate viral progeny.
38. The library of virions according to 37, where each particle contains the genetic information necessary to generate viral progeny.
39. The library of virions according to 36 to 38 generated by using any of the nucleic acids as defined in 1 to 35.
40. The library of virions according to 36 to 39, obtainable by expressing the nucleic acids of the libraries as defined in 1 to 35.
41. A nucleic acid encoding a cap gene comprising at least one recombination site within the cap gene, preferably for the Gateway or cre/lox system preferably after amino acid position 587 of VP1 wherein the inserted nucleic acid sequences are inserted after a nucleic acid corresponding to any site within the first amino-terminal amino acids 1 to 50 of VP1, or corresponding to amino acid positions 261, 381, 447, 534, 573, and/or 587 of VP1, preferably to amino acid position 447 or 587.
42. A nucleic acid encoding a cap gene comprising at least one endonuclease restriction site or polylinker that is not present in the respective wildtype gene and that is inserted after a nucleic acid corresponding to any site within the first amino-terminal amino acids 1 to 50 of VP1, or corresponding to amino acid positions 261, 381, 447, 534, 573, and/or 587 of VP1, preferably to amino acid position 447 or 587.
43. The nucleic acid encoding a cap gene of one of 41 or 42, wherein such recombination site, endonuclease restriction site or polylinker further contains a stop codon.
44. The nucleic acid encoding a cap gene according to any of 41 to 43, wherein such cap gene has at least one mutation leading to for example at least one point mutation, at least one internal deletion, insertion and/or substitution of one or several amino acids or at least one N- or C-terminal deletion, insertion and/or substitution of one or several amino acids, or a combination of these-mutations.
45. The nucleic acid encoding a cap gene according to any of 41 to 44, wherein the cap gene has a further constant insertion of at least one codon upstream and/or downstream of the insertion site of the inserted nucleic acid sequence, preferably of one or two or three codons coding for Ala, Gly, Leu, Ile, Asp and/or Arg, especially an insertion of three Ala upstream and two Ala downstream of the insertion site.
46. A nucleic acid encoding a cap gene with a sequence of the plasmid pWT99oen.
47. A nucleic acid encoding a cap gene, wherein such cap gene has an insertion leading to amino acids comprising an RGD or DDD motif, preferably an RGDXP or DDDXP motif, especially an RGD motif that is not present in human proteins, excluding the insertion AGTFALRGDNPQG.
48. A nucleic acid encoding a cap gene, wherein such cap gene has an insertion leading to amino acids RGDXXXX, RGDXPXX, DDDXPXX, RGDAVGV, . RGDTPTS, RSNAVVP, RDNAVVP, GKLFVDR, GENQARS, RSNGVVP, or NSVRAPP.
49. A nucleic acid encoding a cap gene, wherein such cap gene has an insertion the nucleotidic sequence GANGANNACNNNNCNANNANN (N=A,C,G or T) or an insertion comprising that sequence.
50. The nucleic acid according to any of 47 to 49, wherein the inserted nucleic acid sequences are inserted at any site corresponding to the first amino-terminal amino acids 1 to 50 of VP1, after corresponding amino acid positions 261, 381, 447, 534, 573, and/or 587 of VP1, preferably after amino acid position 447 or 587.
51. The nucleic acid according to any of 47 to 50, wherein such cap gene has at least one mutation leading to for example at least one point mutation, at least one internal deletion, insertion and/or substitution of one or several amino acids or at least one N- or C-terminal deletion, insertion and/or substitution of one or several amino acids, or a combination of these mutations.
52. The nucleic acid according to 47 to 51, wherein the cap gene has a further constant insertion of at least one codon upstream and/or downstream of the insertion site of the inserted nucleic acid sequence, preferably of two or three codons coding for Ala, Gly, Leu, Ile, Asp and/or Arg, preferably an insertion of three Ala upstream and two Ala downstream of the insertion site.
53. Use of a nucleic acid encoding a cap gene according to 41 to 52 for the preparation of a library of nucleic acids comprising a multiplicity of expressible cap genes from at least one eukaryotic virus, preferably a parvovirus.
54. A vector construct comprising a nucleic acid according to any of 41 to 52.
55. A bacterium or a cell comprising a nucleic acid according to any of 41 to 45 and/or a vector construct according to 54.
56. A method for the selection of a recombinant virion with an increased infectivity or specificity for a specific cell type comprising the steps of
   i) providing at least one first cell with a vector construct comprising at least one nucleic acid from the library according to any of 17 to 40 together with ITRs and an gene providing necessary functions of a Rep-protein for-the packaging of a virion;
   ii) providing such first cell with necessary cellular, viral, physical and/or chemical helper functions for the packaging of a virions;
   iii) incubating such first cell under suitable conditions for the packaging of virions and collecting produced virions by such first cell
   iv) infecting at least one second cell with such virions
   v) providing such second cell with necessary cellular, viral, physical and/or chemical helper functions for the packaging of a virion;
   vi) incubating such second cell under suitable conditions for the packaging of virions and collecting produced virions by such second cell;
   whereas steps iv) to vi) can be repeated several times.
57. The method of 56 additionally comprising the steps
   vii) infecting at least one third cell with the collected virions, whereas such third cell is not permissive for such virions, and
   viii) collecting the virions that did not infect such third cell.
58. A method for the selection of a recombinant virion with a modified immunogenicity comprising the steps of
   i) providing at least one first cell with a vector construct comprising at least one nucleic acid from the library of the invention together with a second nucleic acid necessary for the packaging of virion.
   ii) providing such first cell with necessary cellular, viral, physical and/or chemical helper functions for the packaging of virions if necessary;
   iii) incubating such first cell under suitable conditions for the packaging of virions and collecting produced virions by such first cell;
   iv) applying an immunoselection step to the produced virions;
   v) infecting at least one first or second cell with such collected virions;
   vi) providing such first or second cell with necessary cellular, viral, physical and/or chemical helper functions for the packaging of a virion;
   vii) incubating such first or second cell under suitable conditions for the packaging of virions and collecting produced virions by such first or second cell;
   whereas steps iv) to vii) can be repeated several times.
59. The method of 58, whereas the immunoselection step is a preincubation of the produced virions with monoclonal or polyclonal antibodies or an immunodepletion reaction.
60. A method for the identification of a mutant cap gene comprising the steps of 56 or 57 or of 58or 59 and in addition the step of ix) cloning the nucleic acid of the cap gene(s) of the virion.
61. The method according to any of the 56 to 60, comprising in addition a step for selection of virions, e.g. an affinity binding step of virions, an ion exchange chromatography step or an immune-selection step.
62. A method for the selection of a receptor binding motif comprising the steps as defined in 56 to 61, wherein such second cell expresses the respective receptor.
63. The method of 62, wherein such second cell recombinantly expresses or over-expresses such receptor.
64. A method for the in vivo selection of a recombinant virion capable of infecting a specific cell type comprising the steps of
   i) providing at least one first cell with a vector construct comprising at least one nucleic acid from the library according to any of the 17-to 40 together with-ITRs and a gene providing necessary functions of a Rep protein for the packaging of a virion;
   ii) providing such first cell with necessary cellular or viral helper functions for the packaging of a virion;
   iii) incubating such first cell under suitable conditions for the packaging of virions and collecting produced virions by such first cell;
   iv) infecting an animal with such virions.
65. A method for the identification of a mutant cap gene leading to virions having an increased infectivity or specificity for a specific cell type comprising the steps of 64 and in addition the step of
   v) cloning the nucleic acid of the cap gene(s) from such cell type of the animal.
66. A Cap protein, encoded by the nucleic acids according to 47 or 48.
67. A polypeptide comprising a peptide with the sequence RGDAVGV, RGDTPTS, GKLFVDR, RDNAVVP, GENQARS, RSNGVVP, RSNAVVP or NSVRAPP..
68. The polypeptide according to 67, consisting of a peptide with the sequence RGDAVGV, RGDTPTS, GKLFVDR, RDNAVVP, GENQARS, RSNGWP, RSNAWP or NSVRAPP.
69. The polypeptide according to 67 or 68, wherein the polypeptide is a Cap polypeptide, preferably derived from a parvovirus, especially from an AAV.
70. Use of a polypeptide according to 67 to 69 or comprising or consisting of -a- peptide with-the--sequence .RGDXXXX, RGDXPXX,...or... DDDXPXX, with the exception of AGTFALRGDNPQG, for the retargeting of eukaryotic viruses, preferably parvorviruses, especially AAV.
71. A recombinant virion obtainable by the method according to 56 to 59 or 61 to 64.
72. A mutant cap gene obtainable by the method according to 60 to 63 or 65.
73. A Cap protein encoded by a mutant cap gene according to 72.
74. A virion comprising a Cap protein according to 73.
75. A medicament for the treatment of a patient suffering from cancer, an autoimmune disesase, an infectious disease or a genetic defect comprising a virion according to 71 or 74, a cap gene according to 72 or a Cap protein according to 73.
76. A method for treating a patient suffering from cancer, an autoimmune disesase, an infectious disease or a genetic defect comprising administering to the patient a virion according to 71 or 74, a cap gene according to 72 or a Cap protein according to 73.

## Claims

1. A method for the production of a library of nucleic acids comprising a multiplicity of greater than 10⁵, preferably of greater than 10⁶ expressible structural genes from at least one eukaryotic virus, comprising the steps of:
a) providing a set of nucleic acids, each encoding at least one structural gene from a eukaryotic virus and comprising a suitable packaging sequence, wherein the structural gene contains an insert (2) preventing the formation of a functional structural protein, preferably by containing a stop codon,
more preferably wherein the set of nucleic acids is derived from one nucleic acid, and
b) inserting an insert (1) into the structural gene to remove a sequence of the structural gene, wherein the removed sequence comprises or is part of the insert (2) thereby forming potentially functional structural genes,
especially wherein by inserting insert (1) the stop codon is removed.

2. The method of claim 1, wherein the structural genes are from a non-enveloped virus such as parvovirus or adenovirus or an enveloped virus such as retrovirus, lentivirus, or herpes virus, particularly wherein the herpes virus is HSV1, HSV2, EBV, Varizella zoster virus or human herpes virus 1, 2, 3, 4, 7 or 8,
preferably from a parvovirus selected from the group consisting of Adeno-associated Virus (AAV), Canine Parvovirus (CPV), MVM, B19, H1, AAAV or GPV,
especially from an AAV.

3. The method according to claims 1 to 2, wherein the number of nucleotides of insert (1) and/or insert (2), preferably of insert (1) and insert (2), is three or a multiple of three,
preferably wherein insert (1) is randomly or partially randomly generated, especially insert (1) does not contain any stop codons.

4. The method of claims 1 to 3, wherein insert (1) is inserted at a region of the cap gene encoding amino acids on the surface of the structural protein, preferably wherein the virus is AAV and wherein the insert (1) is inserted after a nucleic acid corresponding to any site within the first amino terminal amino acids 1 to 50, or corresponding to the adjacent 5 amino acids of amino acid positions 261, 381, 447, 534, 573, and/or 587 of the capsid protein VP1, preferably to amino acid position 447 or 587.

5. The method of claims 1 to 4, wherein the library has a multiplicity of viral mutants that is greater than 10⁵, especially greater than 10⁶.

6. A library of nucleic acids obtainable by the method of any of claims 1 to 5, wherein the multiplicity of expressible structural genes is greater than 10⁵, preferably greater than 10⁶,
preferably wherein the library has a multiplicity of viral mutants that is greater than 10⁵, especially greater than 10⁶,
more preferably, wherein the nucleic acid further comprises packaging sequences such as AAV ITRs and at least one expressible gene providing necessary functions for replication and packaging of virions such as an AAV Rep protein.

7. The library according to claim 6, wherein the inserted nucleic acid sequences lead to an insertion of amino acids into the VP1, VP2 and/or VP3 structural protein of AAV, preferably at a site that is located on the surface of the capsid.

8. A method of generating a library of virions, especially parvovirus virions, with capsid protein modifications, wherein the library of the nucleic acids of claims 6 to 7 is used in the method.

9. A method for the selection of a recombinant virion with an increased infectivity or specificity for a specific cell type comprising the steps of
i) providing at least one first cell with a vector construct comprising the library of nucleic acids according to the claims 6 to 7 together with ITRs and an gene providing necessary functions of a Rep protein for the packaging of a virion;
ii) providing such first cell with necessary cellular, viral, physical and/or chemical helper functions for the packaging of a virions;
iii) incubating such first cell under suitable conditions for the packaging of virions and collecting produced virions by such first cell;
iv) infecting at least one second cell with such virions;
v) providing such second cell with necessary cellular, viral, physical and/or chemical helper functions for the packaging of a virion; and
vi) incubating such second cell under suitable conditions for the packaging of virions and collecting produced virions by such second cell; and optionally
vii) infecting at least one third cell with the collected virions, whereas such third cell is not permissive for such virions, and
viii) collecting the virions that did not infect such third cell.
whereas steps iv) to vi) can be repeated several times.

10. A method for the selection of a recombinant virion with a modified immunogenicity comprising the steps of
i) providing at least one first cell with a vector construct comprising the library of nucleic acids according to the claims 6 to 7 together with a second nucleic acid necessary for the packaging of virion;
ii) providing such first cell with necessary cellular, viral, physical and/or chemical helper functions for the packaging of virions if necessary;
iii) incubating such first cell under suitable conditions for the packaging of virions and collecting produced virions by such first cell;
iv) applying an immunoselection step to the produced virions;
v) infecting at least one first or second cell with such collected virions;
vi) providing such first or second cell with necessary cellular, viral, physical and/or chemical helper functions for the packaging of a virion; and
vii) incubating such first or second cell under suitable conditions for the packaging of virions and collecting produced virions by such first or second cell;
whereas steps iv) to vii) can be repeated several times,
preferably, whereas the immunoselection step is a preincubation of the produced virions with monoclonal or polyclonal antibodies or an immunodepletion reaction.

11. The method according to the claims 9 to 10, comprising in addition a step for selection of virions, e.g. an affinity binding step of virions, an ion exchange chromatography step or an immuno-selection step.

12. A method for the selection of a receptor binding motif comprising the steps as defined in claims 9 to 11, wherein such second cell expresses the respective receptor,
preferably wherein such second cell recombinantly expresses or over-expresses such receptor.

13. A method for the in vivo selection of a recombinant virion capable of infecting a specific cell type comprising the steps of
i) providing at least one first cell with a vector construct comprising the library of nucleic acids according to the claims 6 to 7 together with ITRs and a gene providing necessary functions of a Rep protein for the packaging of a virion;
ii) providing such first cell with necessary cellular or viral helper functions for the packaging of a virion;
iii) incubating such first cell under suitable conditions for the packaging of virions and collecting produced virions by such first cell; and
iv) infecting an non-human animal with such virions.

14. A method for the identification of a mutant cap gene leading to virions having an increased infectivity or specificity for a specific cell type comprising the steps of claim 13 and in addition the step of v) cloning the nucleic acid of the cap gene(s) from such cell type of the animal.

15. A library of virions, especially parvovirus virions, with capsid protein modifications, wherein the library of virions comprises the library of the nucleic acids of claims 6 to 7.


SEQUENCE LISTING

<110>  MediGene AG

<120>  A library of modified structural genes or capsid modified particles
useful for the identification of viral clones with desired cell tropism

<130>  M38559PC

<140>  PCT/EP02/14750
<141>  2002-12-23

<150>  US60/344131
<151>  2001-12-21

<150>  US60/362349
<151>  2002-03-07

<150>  US60/407116
<151>  2002-08-30

<160>  25

<170>  PatentIn version 3.1

<210>  1
<211>  5
<212>  PRT
<213>  artificial sequence

<220>
<223>  amino acid insertion
<220>
<221>  MISC_FEATURE
<222>  (4)..(4)

<223>  X = any

<400>  1

Arg Gly Asp Xaa Pro
1               5

<210>  2

<211>  5

<212>  PRT

<213>  artificial sequence

<220>

<223>  amino acid insertion

<220>

<221>  MISC_FEATURE

<222>  (4)..(4)

<223>  X = any

<400>  2

Asp Asp Asp Xaa Pro
1               5

<210>  3

<211>  13

<212>  PRT

<213>  artificial sequence

<220>

<223>  amino acid insertion

<400>  3

Ala Gly Thr Phe Ala Leu Arg Gly Asp Asn Pro Gln Gly
1               5                   10

<210>  4

<211>  7

<212>  PRT

<213>  artificial sequence

<220>

<223>  amino acid insertion

<220>

<221>  MISC_FEATURE

<222>  (4)..(7)

<223>  X = any


<400>  4

Arg Gly Asp Xaa Xaa Xaa Xaa
1               5


<210>  5

<211>  7

<212>  PRT

<213>  artificial sequence


<220>

<223>  amino acid insertion

<220>

<221>  MISC_FEATURE

<222>  (4)..(4)

<223>  X = any


<220>

<221>  MISC_FEATURE

<222>  (6)..(7)

<223>  X = any


<400>  5

Arg Gly Asp Xaa Pro Xaa Xaa
1               5


<210>  6

<211>  7

<212>  PRT

<213>  artificial sequence

<220>

<223>  amino acid insertion

<220>

<221>  MISC_FEATURE

<222>  (4)..(4)

<223>  X = any

<220>

<221>  MISC_FEATURE

<222>  (6)..(7)

<223>  X = any

<400>  6

Asp Asp Asp Xaa Pro Xaa Xaa
1               5

<210>  7

<211>  7

<212>  PRT

<213>  artificial sequence

<220>

<223>  amino acid insertion

<400>  7

Arg Gly Asp Ala Val Gly Val
1               5

<210>  8

<211>  7

<212>  PRT

<213>  artificial sequence

<220>

<223>  amino acid insertion

<400>  8

Arg Gly Asp Thr Pro Thr Ser
1               5

<210>  9

<211>  7

<212>  PRT

<213>  artificial sequence

<220>

<223>  amino acid insertion

<400>  9

Gly Lys Leu Phe Val Asp Arg
1               5

<210>  10

<211>  7

<212>  PRT

<213>  artificial sequence

<220>

<223>  amino acid insertion

<400>  10

Arg Asp Asn Ala Val Val Pro
1               5

<210>  11

<211>  7

<212>  PRT

<213>  artificial sequence

<220>

<223>  amino acid insertion

<400>  11

Gly Glu Asn Gln Ala Arg Ser
1               5

<210>  12

<211>  7

<212>  PRT

<213>  artificial sequence

<220>

<223>  amino acid insertion

<400>  12

Arg Ser Asn Gly Val Val Pro
1               5

<210>  13

<211>  7

<212>  PRT

<213>  artificial sequence

<220>

<223>  amino acid insertion

<400>  13

Arg Ser Asn Ala Val Val Pro
1               5

<210>  14

<211>  7

<212>  PRT

<213>  artificial sequence

<220>

<223>  amino acid insertion

<400>  14

Asn Ser Val Arg Ala Pro Pro
1               5

<210>  15

<211>  21

<212>  DNA

<213>  artificial sequence

<220>

<223>  nucleotidic sequence insertion

<220>

<221>  misc_feature

<222>  (3)..(3)

<223>  N = A, C, G or T


<220>

<221>  misc_feature

<222>  (6)..(7)

<223>  N = A, C, G or T


<220>

<221>  misc_feature

<222>  (10)..(13)

<223>  N = A, C, G or T


<220>

<221>  misc_feature

<222>  (15)..(15)

<223>  N = A, C, G or T


<220>

<221>  misc_feature

<222>  (17)..(18)

<223>  N = A, C, G or T


<220>

<221>  misc_feature

<222>  (20)..(21)

<223>  N = A, C, G or T


<400>  15
gangannacn nnncnannan n

<210> 16

<211> 6

<212> PRT

<213> artificial sequence

<220>

<223> peptide

<400> 16

Gly Arg Gly Asp Thr Pro
1               5

<210> 17

<211> 5

<212> PRT

<213> artificial sequence

<220>

<223> peptide

<400> 17

Gly Arg Gly Glu Ser
1               5

<210> 18

<211> 5

<212> PRT

<213> artificial sequence

<220>

<223> peptide

<400> 18

Arg Gly Asp Thr Pro
1               5

<210> 19

<211> 54

<212> DNA

<213> artificial sequence

<220>

<223>  pool of single stranded DNA molecules

<220>

<221>  misc_feature

<222>  (13)..(13)

<223>  V = A/G/C (not T)


<220>

<221>  misc_feature

<222>  (16)..(16)

<223>  V = A/G/C (not T)


<220>

<221>  misc_feature

<222>  (19)..(19)

<223>  V = A/G/C (not T)


<220>

<221>  misc_feature

<222>  (22)..(22)

<223>  V = A/G/C (not T)


<220>

<221>  misc_feature

<222>  (25)..(25)

<223>  V = A/G/C (not T)


<220>

<221>  misc_feature

<222>  (28)..(28)

<223>  V = A/G/C (not T)


<220>

<221>  misc_feature
<222>  (31)..(31)
<223>  V = A/G/C (not T)

<220>
<221>  misc_feature
<222>  (14)..(15)
<223>  N = A/G/C/T

<220>
<221>  misc_feature
<222>  (17)..(18)
<223>  N = A/G/C/T

<220>
<221>  misc_feature
<222>  (20)..(21)
<223>  N = A/G/C/T

<220>
<221>  misc_feature
<222>  (23)..(24)
<223>  N = A/G/C/T

<220>
<221>  misc_feature
<222>  (26)..(27)
<223>  N = A/G/C/T

<220>
<221>  misc_feature
<222>  (29)..(30)
<223>  N = A/G/C/T

<220>

<221>  misc_feature

<222>  (32)..(33)

<223>  N = A/G/C/T


<400>  19
ttggcgcgcc gcvnnvnnvn nvnnvnnvnn vnnggcggcc gcttttttcc ttga          54

<210>  20

<211>  15

<212>  DNA

<213>  artificial sequence


<220>

<223>  primer

<400>  20
ctcaaggaaa aaagc                                                    15

<210>  21

<211>  18

<212>  DNA

<213>  artificial sequence


<220>

<223>  primer 4066Back

<400>  21
atgtccgtcc gtgtgtgg                                                 18

<210>  22

<211>  6

<212>  PRT

<213>  artificial sequence


<220>

<223>  peptide

<400>  22

Gly Arg Gly Asp Thr Pro

1                   5

<210>  23

<211>  4

<212>  PRT

<213>  artificial sequence


<220>

<223>  peptide

<400>  23

Arg Gly Asp Ser
1


<210>  24

<211>  19

<212>  PRT

<213>  artificial sequence


<220>

<223>  pool of randomly generated oligonucleotides

<220>

<221>  MISC_FEATURE

<222>  (7)..(13)

<223>  X = any


<400>  24

Arg Gly Asn Ala Ala Ala Xaa Xaa Xaa Xaa Xaa Xaa Xaa Ala Ala Arg
1               5                   10                  15

Gln Ala Ala


<210>  25

<211>  59

<212>  DNA

<213>  artificial sequence


<220>

<223>  random insertion containing region of cap gene

<400>  25
agaggcaatg cggccgcccg tggcgacgcc gttggggttg cggcgcgcca agcagctac       59